(19) Europäisches Patentamt
European Patent Office
Office européen des brevets

(11) Numéro de publication: **0 533 516 B1**

# (12) FASCICULE DE BREVET EUROPEEN

(45) Date de publication de fascicule du brevet: **08.02.95**

(51) Int. Cl.$^{6}$: **C07D 295/185**, C07D 207/16, A61K 31/495

(21) Numéro de dépôt: **92402309.6**

(22) Date de dépôt: **20.08.92**

(54) **Nouveaux composés amidiques des 1-(alcoxybenzyl) pipérazines, leurs procédés de préparation, et les compositions pharmaceutiques qui les contiennent.**

(30) Priorité: **20.08.91 FR 9110431**

(43) Date de publication de la demande:
**24.03.93 Bulletin 93/12**

(45) Mention de la délivrance du brevet:
**08.02.95 Bulletin 95/06**

(84) Etats contractants désignés:
**AT BE CH DE DK ES FR GB GR IE IT LI LU NL PT SE**

(56) Documents cités:
**FR-A- 2 503 709**
**GB-A- 2 185 483**

**DATABASE WPIL Week 9013, Derwent Publications Ltd., London, GB; AN 90-094578**

(73) Titulaire: **ADIR ET COMPAGNIE**
**1 rue Carle Hébert**
**F-92415 Courbevoie Cédex (FR)**

(72) Inventeur: **Gaudry, Michel**
**Impasse du Puits,**
**rue de la fontaine**
**F-78330 Fontenay le Fleury (FR)**
Inventeur: **Pfeiffer, Bruno**
**6 rue Jean Thomas**
**F-95600 Eaubonne (FR)**
Inventeur: **Renard, Pierre**
**50 avenue de Villeneuve l'Etang**
**F-78000 Versailles (FR)**
Inventeur: **Renaud de la Faverie, Jean-François**
**7 rue des Erables,**
**Rocquencourt**
**F-78150 Le Chesnay (FR)**
Inventeur: **Adam, Gérard**
**9 Clos du Mesnil,**
**route du Pecq**
**F-78600 Le Mesnil le Roi (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

L'invention concerne de nouveaux composés amidiques des 1-(alcoxybenzyl)pipérazines, leurs procédés de préparation et les compositions pharmaceutiques qui les contiennent.

On connait dans la littérature de nombreux composés amidiques des 1-(alcoxybenzyl)pipérazines.

Ainsi certains composés de formule générale (a) :

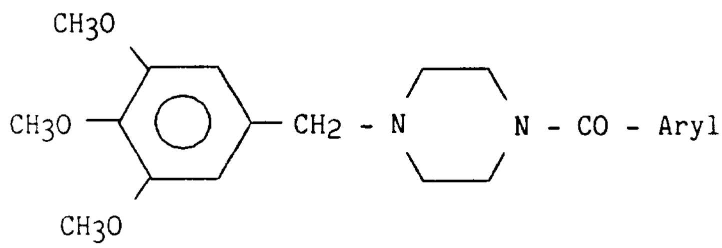

(a)

sont décrits comme antagonistes du Platelet Activating Factor ou P.A.F. (EP 318 235, EP 284 359).

D'autres composés comportant un groupement oxo-4 quinol-3 yl possèdent des propriétés anti-inflammatoires (JP 8 961 461),
et des composés possédant un groupement benzamidique sont présentés comme inhibiteurs de l'anhydrase carbonique (EP 171 636).

On note également que certains cinnamides de 1-(alcoxybenzyl)pipérazines sont décrits comme antihypertenseurs (US 436 8199).

Structurellement plus proches des composés de l'invention, on connait dans la littérature des dérivés pyroglutamidiques de la 1-(p-méthoxybenzyl)pipérazine décrits comme possédant une activité nootrope (DE 3 701 494).

La demande de brevet JP (A) 2045464 décrit des dérivés de la pyrrolidinone présentés comme utiles pour l'amélioration des dysfonctions cérébrales.

On connait également des composés de formule (b) :

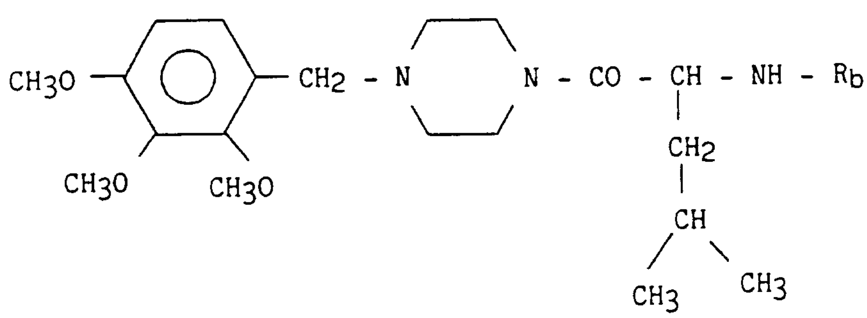

(b)

dans laquelle $R_b$ représente un groupe protecteur pour la fonction amine d'un aminoacide, pour lequels aucune activité pharmacologique n'a été mentionnée et qui sont utilisés comme intermédiaires de synthèse de composés caractérisés par la présence d'un cycle oxirane et présentés notamment comme inhibiteurs d'enzymes protéolytiques (Biochemical Pharmacology 1985 ; vol 34 (n° 21) : 3875 - 3880 et Arzneim - Forsch - 1986 ; vol 36 (n° 4) : 671 - 675).

La demanderesse a présentement découvert de nouveaux composés amidiques d'alcoxybenzyl pipérazines qui possèdent une action antiischémique et antianoxique, tant au niveau cérébral qu'au niveau périphérique, nettement supérieure à celle des 1-(alcoxybenzyl)pipérazines les plus proches comme la trimétazidine ou 1-(2,3,4-triméthoxybenzyl) pipérazine.

Plus spécifiquement, l'invention concerne les composés de formule générale (I) :

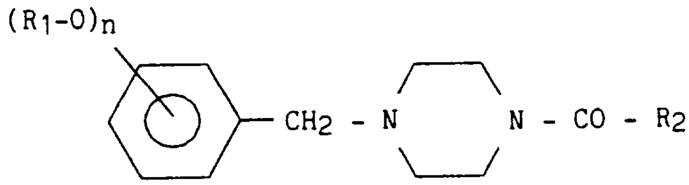

(I)

dans laquelle :

- n, nombre entier, peut prendre les valeurs 1, 2, ou 3,
- $R_1$ est un alkyle linéaire ou ramifié contenant de 1 à 4 atomes de carbone,
- $R_2$ représente :
  un groupement

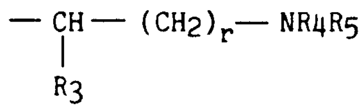

dans lequel :

- r nombre entier peut prendre les valeurs 0, 1, 2,
- $R_3$ représente :
  . a) un atome d'hydrogène,
  . b) un alkyle linéaire ou ramifié de 1 à 6 atomes de carbone éventuellement substitué par :
    * un ou deux groupements choisis parmi : - COOH, $-CO-R_6$, $-CO-O-R_6$ avec $R_6$ étant un groupement
      choisi parmi : alkyle linéaire ou ramifié saturé, insaturé, ou polyinsaturé de 1 à 12 atomes de carbone, et $-(CH_2)_m$-aryle éventuellement substitué où m, nombre entier, peut prendre les valeurs 0, 1, 2, ou 3,
    * ou par un groupement choisi parmi :
      i) -OH, $-O-R_6$, $-O-CO-R_6$, ou $-O-CO-O-R_6$ avec $R_6$ tel que défini précédemment,
      ii) $-NR_7R_8$ avec $R_7$ et $R_8$, identiques ou différents, représentant chacun indépendamment l'un de l'autre un atome d'hydrogène, un alkyle linéaire ou ramifié de 1 à 6 atomes de carbone, $-CO-R_6$ ou $-CO-O-R_6$ avec $R_6$ ayant la même signification que précédemment, et $-(CH_2)_m$-aryle éventuellement substitué, où m nombre entier peut prendre les valeurs 0, 1, 2, ou 3,
      iii) $-CO-NR_9R_{10}$ avec $R_9$ et $R_{10}$, identiques ou différents, ayant la même définition que $R_6$ avec $R_6$ tel que défini précedemment, ou pouvant également représenter un atome d'hydrogène, ou $R_9$ et $R_{10}$ forment ensemble, avec l'atome d'azote qui les porte, un système cyclique de 5 à 7 sommets pouvant comprendre éventuellement un second hétéroatome choisi parmi oxygène, azote, et soufre,
      iv) -SH, ou -SeH,
      v) un groupement $-S-R_6$, $-Se-R_6$, $S-CO-O-R_6$, $-S-S-R_6$ ou $-Se-Se-R_6$ avec $R_6$ tel que défini précédemment,
      vi) un radical guanidino non substitué ou substitué par 1 à 2 groupements choisis parmi nitro et $-CO-O-R_6$ avec $R_6$ tel que défini précédemment,
      vii) un groupement indole ou imidazole éventuellement substitué,
  . c) un groupement $-(CH_2)_m$-phényle éventuellement substitué où m, nombre entier, peut prendre les valeurs 0, 1, 2, ou 3,
  étant entendu que $R_3$ ne peut pas représenter un groupement isobutyle,
- $R_4$ représente un atome d'hydrogène, un alkyle linéaire ou ramifié de 1 à 6 atomes de carbone, un groupement $-(CH_2)_p$-aryle éventuellement substitué où p, nombre entier, peut prendre les valeurs 0, 1, 2, ou 3, ou un groupement amidino non substitué ou substitué par un groupement nitro ou $-CO-O-R_6$ avec $R_6$ tel que défini précédemment, ou $R_4$ forme avec $R_3$ et les atomes auxquels ils sont liés un système mono ou bicyclique choisi parmi : pyrrolidine, pipéridine, perhydroindole, indoline, 2-aza bicyclo[2, 2, 2]octane, et 2-aza bicyclo[3, 3, 0]octane, ces systèmes cycliques pouvant être éventuellement substitués par un ou plusieurs groupements choisis parmi :

hydroxy, oxo, alkyle linéaire ou ramifié de 1 à 6 atomes de carbone, alcoxy linéaire ou ramifié de 1 à 6 atomes de carbone,

avec la réserve que $R_3$ et $R_4$ ne peuvent pas former ensemble, avec les atomes auxquels ils sont liés, une pyrrolidine substituée par un groupement oxo en $\alpha$ de l'azote,

- $R_5$ représente :
  - un atome d'hydrogène,
  - un groupement $-(CH_2)_q$-aryle éventuellement substitué où q, nombre entier, peut prendre les valeurs 0, 1, 2, ou 3,
  - une chaine alkyle de 1 à 20 atomes de carbone, linéaire ou ramifiée, saturée, insaturée ou polyinsaturée, éventuellement interrompue par un ou plusieurs atomes d'oxygène, de soufre, ou d'azote, et éventuellement substituée par un ou plusieurs radicaux hydroxy, amino, alkylamino linéaire ou ramifié de 1 à 6 atomes de carbone, ou alcoxy linéaire ou ramifié de 1 à 6 atomes de carbone,
  - un groupement $-CO-R_{11}$ ou $-CO-O-R_{11}$ où $R_{11}$ représente :
    . une chaine alkyle de 1 à 20 atomes de carbone, linéaire ou ramifiée, saturée, insaturée ou polyinsaturée, éventuellement interrompue par un ou plusieurs atomes d'oxygène, de soufre, ou d'azote, et éventuellement substituée par un ou plusieurs radicaux hydroxy, amino, alkylamino linéaire ou ramifié de 1 à 6 atomes de carbone, alcoxy linéaire ou ramifié de 1 à 6 atomes de carbone, guanidino, ou guanidino substitué par 1 à 2 groupements choisis parmi nitro et $-CO-O-R_6$ avec $R_6$ tel que défini précédemment,
    . un groupement $-(CH_2)_q$-aryle éventuellement substitué où q, nombre entier, peut prendre les valeurs 0, 1, 2, ou 3,

étant entendu que, sauf précisions contraires, :

le terme "aryle" désigne les radicaux phényle et naphtyle,

le terme "substitué" associé aux expressions "$(CH_2)_m$-phényle", "$-(CH_2)_m$-aryle", "$-(CH_2)_p$-aryle", "$-(CH_2)_q$-aryle", "imidazole", et "indole" signifie que ces radicaux peuvent être substitués sur le cycle par un ou plusieurs groupements choisis parmi : hydroxy, halogène, nitro, trifluorométhyle, alkyle linéaire ou ramifié de 1 à 6 atomes de carbone, alcoxy linéaire ou ramifié de 1 à 6 atomes de carbone, $-(CH_2)_t$-phényle, $-O-(CH_2)_t$-phényle, et $O-CO-O-(CH_2)_t$-phényl, où t, nombre entier, peut prendre les valeurs 0, 1, 2, ou 3,

- leurs isomères, diastéréoisomères, épimères, isolés ou sous forme de mélange ainsi que, le cas échéant, leurs sels d'addition à un acide ou à une base pharmaceutiquement acceptable.

La présente invention a également pour objet le procédé de préparation des composés de formule (I), caractérisé en ce que l'on condense, en utilisant les techniques de couplage passant par la formation d'un ester activé (hydroxy succinimide, hydroxybenzotriazole, en présence de dicyclohexyl carbodiimide), un composé de formule (II) :

$HOOC - R'_2$ (II)

dans laquelle $R'_2$ a la même signification que $R_2$ dans la formule générale (I) avec la réserve que lorsque $R'_2$ représente un groupement

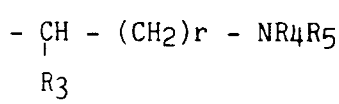

tel que défini dans la formule générale (I) et

que $R_4$ représente un atome d'hydrogène, alors $R_5$ représente un groupement $-CO-R_{11}$ ou $-CO-O-R_{11}$ avec $R_{11}$ ayant la même signification que dans la formule générale (I),

avec une (alcoxybenzyl)pipérazine de formule (III) :

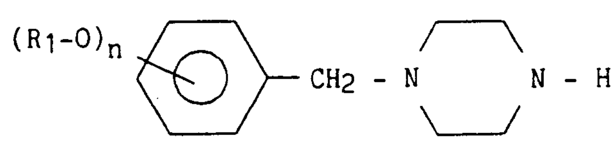

(III)

dans laquelle $R_1$ et n sont tels que définis dans la formule (I) afin d'obtenir un composé de formule (I/a) :

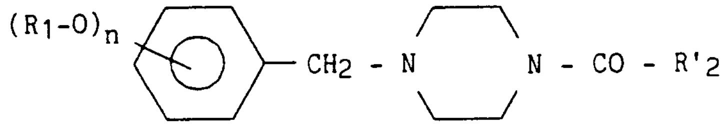 (I/a)

dans laquelle $R_1$, $R'_2$ et n sont tels que définis précédemment,
composés de formule (I/a) qui sont, dans le cas où $R_5$ représente un groupement $-CO-O-R_{11}$, éventuellement soumis si on le désire, aux réactions de déprotection des fonctions aminées en synthèse peptidique (traitement acide ou hydrogénation catalytique suivant la nature de $R_{11}$) afin d'accéder aux composés de formule (I) pour lesquels $R_5$ représente un hydrogène,
les composés de formule (I) pour lesquels $R_5$ représente un atome d'hydrogène pouvant ensuite, le cas échéant, être mis en réaction avec un composé de formule (IV) :

$HOOC-R_{11}$ (IV)

dans laquelle $R_{11}$ est tel que défini précédemment, afin d'obtenir un composé de formule (I/b) :

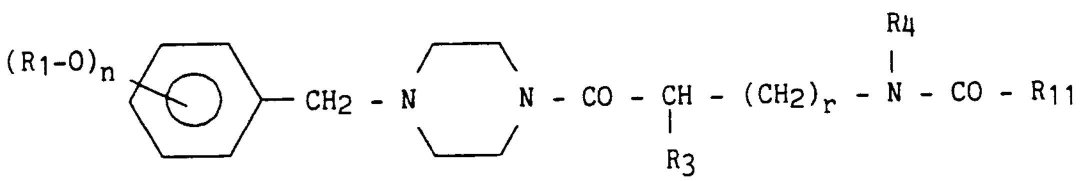 (I/b)

dans laquelle $R_1$, $R_3$, $R_4$, $R_{11}$ et n sont tels que définis précédemment,
cas particulier des composés de formule (I) dans lesquels $R_2$ représente un groupement de formule :

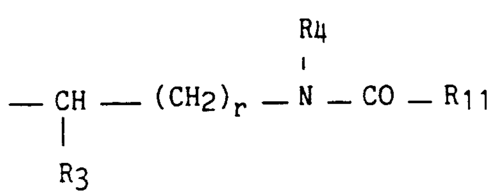

les composés de formule (I) pouvant, si nécessaire, être purifiés, séparés, le cas échéant, en leurs différents isomères optiques, et pouvant également être salifiés, soit avec un acide pharmaceutiquement acceptable, soit, si cela est possible et si on le désire, avec une base pharmaceutiquement acceptable.

Les matières premières utilisées dans le procédé précédemment décrit sont

- soit commerciales,
- soit aisément accessibles à l'homme de l'art selon des procédés décrits dans la littérature.

Comparés à la trimétazidine, les composés de la présente invention présentent tant in vivo que in vitro une activité protectrice cellulaire très largement supérieure, notamment au niveau cérébral.

Les tests réalisés in vitro, sur des astrocytes de rats en culture, placés en condition hypoxique, ont montré que les composés de la présente invention protégeaient de façon remarquable, et bien supérieure à celle de la trimétazidine, l'intégrité cellulaire de ces astrocytes.

D'autres tests effectués in vivo chez la gerbille ont montré que les composés de l'invention augmentaient de façon significative et largement supérieure à celle de la trimétazidine la survie des aminaux chez lesquels on provoque une ischémie cérébrale par ligature de la carotide gauche et retardaient de façon importante l'apparition des premières suffocations chez des animaux que l'on place en condition d'hypoxie normobare.

Les remarquables propriétés des composés de la présente invention les rendent précieux notamment au niveau cérébral pour le traitement et la prévention de l'ischémie cérébrale, de l'hypoxie vasculaire cérébrale, de l'anoxie vasculaire cérébrale et des oedèmes cérébraux.

Plus généralement, le pouvoir protecteur des composés de la présente invention leur confère une utilité dans le traitement des accidents vasculaires cérébraux, des traumatismes crâniens, des encéphalopathies, des maladie neuro-dégénératives, et des troubles de la sénéscence.

La capacité des composés de l'invention à protéger les cellules au cours d'une hypoxie permet également leur utilisation dans le traitement et la prévention de l'ischémie de type périphérique, en cardiologie : l'ischémie myocardique et l'ischémie coronarienne et leurs différentes expressions cliniques : angine de poitrine, infarctus du myocarde, troubles du rythme, spasme vasculaire, insuffisance cardiaque, ainsi qu'en ophtamologie et en oto-rhino-laryngologie lors d'atteintes vasculaires chorio-rétiniennes, de vertiges d'origine vasculaire, de vertiges de Ménière ou d'acouphènes.

L'invention concerne également les sels d'addition des composés de formule (I) obtenus avec une base ou un acide minéral ou organique, pharmaceutiquement acceptable.

Parmi les acides pharmaceutiquement acceptables que l'on peut utiliser pour former un sel d'addition avec les composés de l'invention, on peut citer, à titre d'exemple et de façon non limitative, les acides chlorhydrique, sulfurique, phosphorique, tartrique, malique, maléique, fumarique, oxalique, méthanesulfonique, éthanesulfonique, camphorique, et citrique.

Comme bases pharmaceutiquement acceptables pouvant salifier les composés de formule (I) on pourra citer à titre d'exemples et de façon non limitative, les hydroxydes de sodium, de potassium, de calcium ou d'aluminium, les carbonates de métaux alcalins ou alcalinoterreux ou parmi les bases organiques : la triéthylamine, la benzylamine, la diéthanolamine, la tert-butylamine, la dicyclohexylamine, l'arginine.

L'invention s'étend aussi aux compositions pharmaceutiques renfermant comme principe actif un composé de formule générale (I) ou un de ses sels d'addition, avec une base ou un acide, minéral ou organique, pharmaceutiquement acceptable, en association avec un ou plusieurs excipients inertes, non toxiques convenant pour l'usage pharmaceutique et/ou un agent liant, un agent aromatisant, un agent de délitement, un agent édulcorant, un agent lubrifiant ou bien encore un véhicule liquide adapté à l'administration par voie intraveineuse, tel que l'eau stérile apyrogène.

Parmi les compositions pharmaceutiques selon l'invention, on pourra citer, à titre d'exemples et de façon non limitative, plus particulièrement celles qui conviennent pour l'administration orale, parentérale, oculaire, per ou transcutanée, nasale, rectale perlinguale, ou respiratoire et notamment les préparations injectables, les aérosols, les gouttes oculaires ou nasales, les comprimés, les comprimés sublinguaux, les gélules, capsules, tablettes, glossettes, suppositoires, crèmes, pommades, et les gels.

Les compositions ainsi obtenues se présentent généralement sous forme dosée et peuvent contenir selon les affections traitées, l'âge et le sexe du malade, de 0,1 à 500 mg de principe actif.

Elles peuvent selon le cas être administrées par voie orale, rectale ou parentérale à la dose de 0,1 à 500 mg de une à plusieurs fois par jour.

Les exemples suivants illustrent l'invention.

**EXEMPLE 1 : 1-(2,3,4-TRIMETHOXYBENZYL) 4-[S-BENZYL N-BENZYLOXYCARBONYL L-CYSTEINYL] PIPERAZINE**

**Stade A : N-[S-benzyl N-benzyloxycarbonyl L-cystéinyl]oxy succinimide.**

Ajouter 29,4 mmoles de N-hydroxysuccinimide en solution dans 30 $cm^3$ de chlorure de méthylène, à 29,4 mmoles de S-benzyl N-benzyloxycarbonyl L-cystéine en solution dans 20 $cm^3$ de chlorure de méthylène. Refroidir à 10 °C, puis ajouter 29,4 mmoles de dicyclohexylcarbodiimide.

Agiter pendant 4 heures à -10 ° C puis pendant 1 heure à 20 °C.

La dicyclohexylurée formée est éliminée par filtration et la solution de N-[S-benzyl N-benzyloxycarbonyl L-cystéinyl]oxy succinimide est utilisée telle qu'elle dans l'étape suivante .

**Stade B : 1-(2,3,4-triméthoxybenzyl) 4-[S-benzyl N-benzyloxycarbonyl L-cysteinyl] pipérazine**

La solution chlorométhylènique de N-[S-benzyl N-benzyloxycarbonyl L-cystéinyl]oxy succinimide obtenue au stade précédent est ajoutée à une solution préalablement préparée, et maintenue à 4 ° C, de 29,4 mmoles de 1-(2,3,4-triméthoxybenzyl) pipérazine dans 40 $cm^3$ de chlorure de méthylène.

Après 3 heures d'agitation à 4 ° C et 15 heures d'agitation à 20 ° C , le milieu est mis à sec sous pression réduite et purifié par chromatographie sur colonne de silice (éluant : chloroforme/méthanol (99/1) puis chloroforme/méthanol (98/2)).

Après mise à sec des fractions collectées,on obtient la 1-(2,3,4-triméthoxybenzyl) 4-[S-benzyl N-benzyloxycarbonyl L-cystéinyl] pipérazine.

Pouvoir rotatoire (c = 1, éthanol absolu)
$[\alpha]_D^{20}$ = -21,7°
Caractéristiques spectrales :
Infrarouge ($CHCl_3$, 5 mg/$cm^3$, 0,1 mm) en $cm^{-1}$ : 3000, 2920, 1705, 1635, 1485, 1460, 1250, et 1090.
RMN $^1H$ ($CDCl_3$, référence interne : tétraméthysilane)

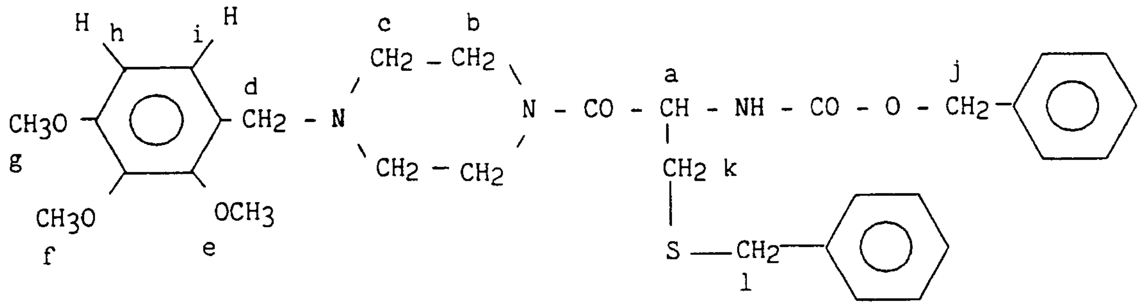

| | | | |
|---|---|---|---|
| 2,30 - 2,80 ppm | triplet | 4H | c |
| 3,30 - 3,70 ppm | triplet | 4H | b |
| 3,75 ppm | singulet | 2H | d |
| 3,89 ppm | 2 singulets | 9H | e, f, g |
| 4,78 ppm | multiplet | 1H | a |
| 5,12 ppm | singulet | 2H | j |
| 5,32 ppm | singulet | 2H | l |
| 5,70 ppm | doublet | 1H | -NH- |
| 6,65 et 6,95 ppm | doublet | 2H | h, i (J = 10$H_z$) |
| 7,22 ppm | multiplet | 5H | phényle |
| 7,33 ppm | multiplet | 5H | phényle |

**Stade C : chlorhydrate de 1-(2,3,4-triméthoxybenzyl) 4-[S-benzyl N-benzyloxycarbonyl L-cysteinyl] pipérazine**

La base obtenue au stade B est mise en suspension dans 200 $cm^3$ d'eau. Après acidification avec de l'acide chlorhydrique jusqu'à dissolution totale (PH : 2-3), la solution obtenue est mise à sec par lyophilisation.

On obtient ainsi le chlorhydrate de 1-(2,3,4-triméthoxybenzyl) 4-[S-benzyl N-benzyloxycarbonyl L-cysteinyl] pipérazine sous forme de cristaux blancs.
Pouvoir rotatoire ( c = 1, éthanol absolu)
$[\alpha]_D^{20}$ = -11,8°

**EXEMPLE 2 : 1-(2,3,4-TRIMETHOXYBENZYL) 4-[N-BENZYLOXYCARBONYL L-PROLYL] PIPERAZINE**

En procédant comme dans l'exemple 1, mais en remplaçant au stade A la S-benzyl N-benzyloxycarbonyl L-cystéine par la N-benzyloxycarbonyl L-proline, on obtient le chlorhydrate de 1-(2,3,4-triméthoxybenzyl) 4-[N-benzyloxycarbonyl L-prolyl] pipérazine.
Point de fusion (base) : 117° C
Pouvoir rotatoire (c : 1, éthanol absolu) (base) :
$[\alpha]_D^{20}$ = -4,4°
Pouvoir rotatoire (C : 1, eau) (chlorhydrate) :
$[\alpha]_D^{20}$ = -8,9°
Caractéristiques spectrales (base) :
Infrarouge ($CHCl_3$ ; 5 mg/$cm^3$ ; 0,1 mm) en $cm^{-1}$ : 3000, 2930, 1690, 1645, 1490, 1465, 1415, 1090.
RMN $^1H$ ($CDCl_3$ ; référence interne : tetraméthylsilane)

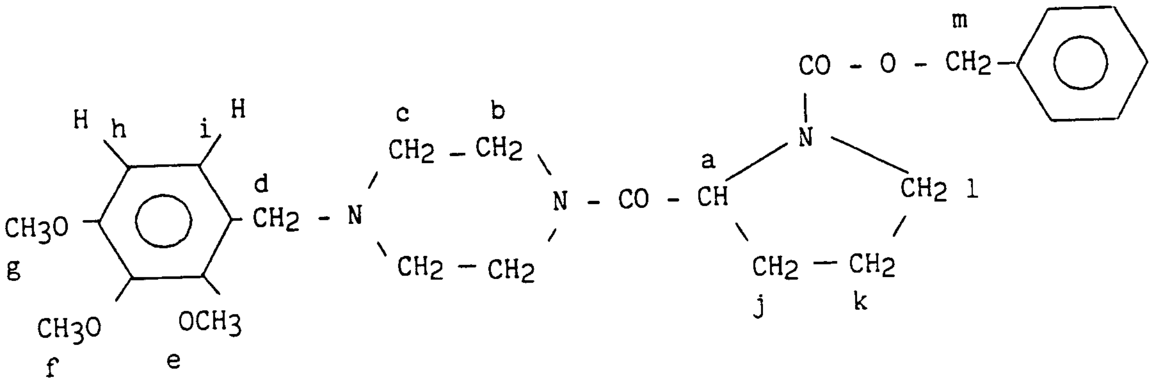

| | | | |
|---|---|---|---|
| 1,8 - 2,6 ppm | multiplet | 8H | c, j, k |
| 3,3 - 3,8 ppm | multiplet | 8H | b, d, l |
| 3,89 ppm | 2 singulets | 9H | e, f, g |
| 4,7 ppm | multiplet | 1H | a |
| 5,12 ppm | | 2H | m |
| 6,66 et 6,96 ppm | doublet | 2H | h, i (J = $9H_z$) |
| 7,35 ppm | multiplet | 5H | phényle |

**EXEMPLE 3 : 1-(2,3,4-TRIMETHOXYBENZYL) 4-(L- PROLYL) PIPERAZINE**

Une solution de 3 grammes de 1-(2,3,4-triméthoxybenzyl) 4-[N-benzyloxycarbonyl L-prolyl] pipérazine dans 100 $cm^3$ de méthanol est hydrogénée pendant 1 heure sous une pression de 1 bar d'hydrogène, en présence de palladium sur charbon à 10 %.

Après élimination du catalyseur par filtration et mise à sec du milieu réactionnel, on obtient la 1-(2,3,4-triméthoxybenzyl) 4-(L-prolyl) pipérazine sous forme d'huile avec un rendement quantitatif de 100 %.

Cette huile peut être reprise dans de l'eau pour donner après acidification avec de l'acide chlorhydrique 1N et isolement par lyophilisation le dichlorhydrate de 1-(2,3,4-triméthoxybenzyl) 4-(L-prolyl) pipérazine.

**EXEMPLE 4 : 1-(2,3,4-TRIMETHOXYBENZYL) 4-[N-BENZYLOXYCARBONYL S-ETHYLTHIO L-CYSTEINYL] PIPERAZINE**

En procédant comme dans l'exemple 1, mais en remplaçant la S-benzyl N-benzyloxycarbonyl L-cystéine par la S-éthylthio N-benzyloxycarbonyl L-cystéine, on obtient le chlorhydrate de 1-(2,3,4-triméthoxybenzyl) 4-[N-benzyloxycarbonyl S-éthylthio L-cystéinyl] pipérazine.

**EXEMPLE 5 : 1-(2,3,4-TRIMETHOXYBENZYL) 4-[S-ETHYLTHIO L-CYSTEINYL] PIPERAZINE**

En procédant comme dans l'exemple 3, mais en remplaçant la 1-(2,3,4-triméthoxybenzyl) 4-[N-benzyloxycarbonyl L-prolyl]pipérazine par la 1-(2,3,4-triméthoxybenzyl) 4-[N-benzyloxycarbonyl S-éthylthio L-cystéinyl] pipérazine, on obtient le dichlorhydrate de 1-(2,3,4-triméthoxybenzyl) 4-[S-éthylthio L-cystéinyl] pipérazine.

**EXEMPLE 6 : 1-(2,3,4-TRIMETHOXYBENZYL) 4- [N-BENZYLOXYCARBONYL L-ALANYL] PIPERAZINE**

En procédant comme dans l'exemple 1, mais en remplaçant la S-benzyl N-benzyloxycarbonyl L-cystéine par la N-benzyloxycarbonyl L-alanine, on obtient le chlorhydrate de 1-(2,3,4-triméthoxybenzyl) 4-(N-benzyloxycarbonylL-alanyl] pipérazine.

Pouvoir rotatoire (c = 1, éthanol absolu) (base) :
$[\alpha]_D^{20}$ = -8,4 °
Pouvoir rotatoire (c = 1, eau) (chlorhydrate) :
$[\alpha]_D^{20}$ = 1 °

Caractéristiques spectrales (base) :

Infrarouge ($CHCl_3$ ; 5 mg/cm$^3$ ; 0,1 mm) en cm$^{-1}$ : 3060, 2990, 1710, 1640, 1490, 1465, 1445, 1095.

RMN $^1H$ ($CDCl_3$, référence interne : tétraméthylsilane)

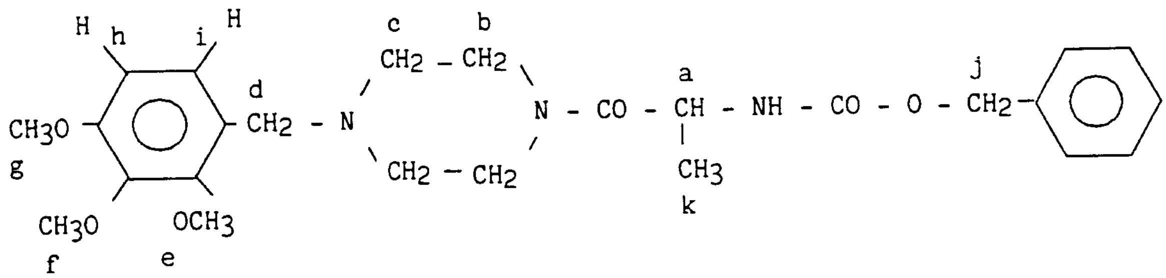

| | | | |
|---|---|---|---|
| 1,09 - 1,18 ppm | doublet | 3H | k |
| 2,46 ppm | multiplet | 4H | c |
| 3,60 ppm | multiplet | 6H | b, d |
| 3,91 ppm | singulets | 9H | e, f, g |
| 4,63 ppm | multiplet | 1H | a |
| 5,11 ppm | singulet | 2H | j |
| 5,85 ppm | doublet | 1H | -NH- |
| 6,65 et 6,95 ppm | doublet | 2H | h, i (J = 9 Hz) |
| 7,32 ppm | multiplet | 5H | phényl |

**EXEMPLE 7 : 1-(2,3,4-TRIMETHOXYBENZYL) 4-(L-ALANYL) PIPERAZINE**

En procédant comme dans l'exemple 3, mais en remplaçant la 1-(2,3,4-triméthoxybenzyl) 4-[N-benzyloxycarbonyl L-prolyl] pipérazine par la 1-(2,3,4-triméthoxybenzyl) 4-[N-benzyloxycarbonyl L-alanyl] pipérazine, on obtient le dichlorhydrate de 1-(2,3,4-triméthoxybenzyl) 4-(L-alanyl) pipérazine.

**EXEMPLE 8 : 1-(2,3,4-TRIMETHOXYBENZYL) 4-[N-BENZYLOXYCARBONYL D-ALANYL] PIPERAZINE**

En procédant comme dans l'exemple 1, mais en remplaçant la S-benzyl N-benzyloxycarbonyl L-cystéine par la N-benzyloxycarbonyl D-alanine, on obtient le chlorhydrate de 1-(2,3,4-triméthoxybenzyl) 4-[N-benzyloxycarbonylD-alanyl] pipérazine.

Pouvoir rotatoire (c = 1, éthanol absolu) :

$[\alpha]_D^{20}$ base = + 8,1 °

**EXEMPLE 9 : 1-(2,3,4-TRIMETHOXYBENZYL) 4-(D-ALANYL) PIPERAZINE**

En procédant comme dans l'exemple 3, mais en remplaçant la 1-(2,3,4-triméthoxybenzyl) 4-[N-benzyloxycarbonyl L-prolyl] pipérazine par la 1-(2,3,4-triméthoxybenzyl) 4-[N-benzyloxycarbonyl D-alanyl] pipérazine, on obtient le dichlorhydrate de 1-(2,3,4-triméthoxybenzyl) 4-(D-alanyl) pipérazine.

**EXEMPLE 10 : 1-(2,3,4-TRIMETHOXYBENZYL) 4-[N-BENZYLOXYCARBONYL L-PHENYLALANYL] PIPERAZINE**

En procédant comme dans l'exemple 1, mais en remplaçant la S-benzyl N-benzyloxycarbonyl L-cystéine par la N-benzyloxycarbonyl L-phénylalanine, on obtient le chlorhydrate de 1-(2,3,4-triméthoxybenzyl) 4-[N-benzyloxycarbonyl L-phénylalanyl] pipérazine.

Pouvoir rotatoire (c = 1, éthanol absolu) :

$[\alpha]_D^{20}$ (base) = + 3,0 °

$[\alpha]_D^{20}$ (chlorhydrate) = + 9,9 °

Caractéristiques spectrales (base) :

Infrarouge ($CHCl_3$ ; 5 mg/$cm^3$ ; 0,1 mm) en $cm^{-1}$ : 2980, 2900, 1710, 1635, 1490, 1460, 1090.
RMN $^1H$ ($CDCl_3$, référence interne : tétraméthylsilane)

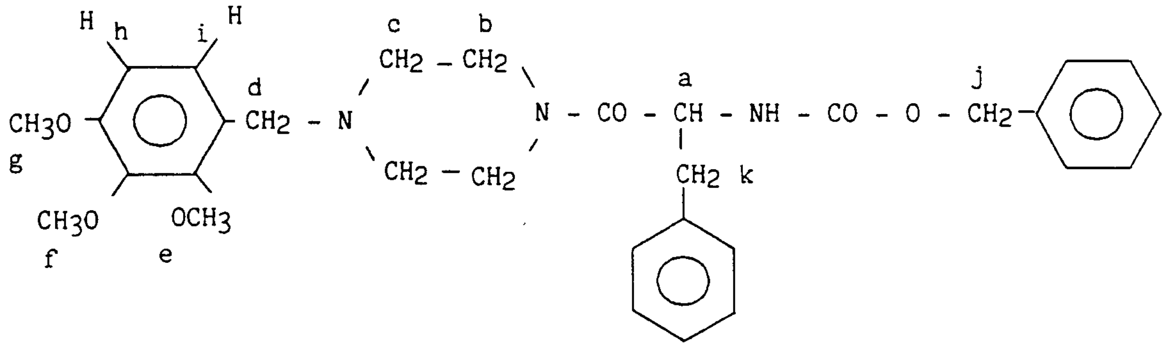

| | | | |
|---|---|---|---|
| 2,2 - 2,5 ppm | multiplet | 4H | c |
| 3,39 ppm | singulet | 2H | d |
| 3,40 - 3,60 ppm | multiplet | 4H | b |
| 3,88 ppm | 2 singulets | 9H | e, f, g |
| 4,89 ppm | multiplet | 1H | a |
| 5,11 ppm | singulet | 2H | j |
| 5,70 ppm | doublet | 1H | -NH- |
| 6,64 et 6,90 ppm | doublet | 2H | h, i ($J = 8H_z$) |
| 7,12 - 7,34 ppm | multiplet | 10H | phényl |

**EXEMPLE 11 : 1-(2,3,4-TRIMETHOXYBENZYL) 4-(L-PHENYLALANYL) PIPERAZINE**

En procédant comme dans l'exemple 3, mais en remplaçant la 1-(2,3,4-triméthoxybenzyl) 4-[N-benzyloxycarbonyl L-prolyl] pipérazine par la 1-(2,3,4-triméthoxybenzyl) 4-[N-benzyloxycarbonyl L-phénylalanyl] pipérazine, on obtient le dichlorhydrate de 1-(2,3,4-triméthoxybenzyl) 4-(L-phénylalanyl) pipérazine.

**EXEMPLE 12 : 1-(2,3,4-TRIMETHOXYBENZYL) 4-[N-BENZYLOXYCARBONYL GLYCYL] PIPERAZINE**

En procédant comme dans l'exemple 1, mais en remplaçant au stade A la S-benzyl N-benzyloxycarbonyl L-cystéine par la N-benzyloxycarbonyl glycine, on obtient le chlorhydrate de 1-(2,3,4-triméthoxybenzyl) 4-[N-benzyloxycarbonyl glycyl] pipérazine.

**EXEMPLE 13 : 1-(2,3,4-TRIMETHOXYBENZYL) 4-GLYCYL PIPERAZINE**

En procédant comme dans l'exemple 3, mais en remplaçant la 1-(2,3,4-triméthoxybenzyl) 4-[N-benzyloxycarbonyl L-prolyl] pipérazine par la 1-(2,3,4-triméthoxybenzyl) 4-[N-benzyloxycarbonyl glycyl] pipérazine, on obtient le dichlorhydrate de 1-(2,3,4-triméthoxybenzyl) 4-glycyl pipérazine.

**EXEMPLES 14 A 46**

En procédant comme dans l'exemple 1, mais en remplaçant au stade A la S-benzyl N-benzyloxycarbonyl L-cystéine par :

. la N-benzyloxycarbonyl L-valine, on obtient :
**EXEMPLE 14 : LA 1-(2,3,4-TRIMETHOXYBENZYL) 4-[N-BENZYLOXYCARBONYL L-VALYL] PIPERAZINE**

. l'acide N-benzyloxycarbonyl 4-aminobutyrique, on obtient :
**EXEMPLE 15 : LA 1-(2,3,4-TRIMETHOXYBENZYL) 4-[N-BENZYLOXYCARBONYL 4-AMINOBUTYRYL] PIPERAZINE**

. l acide N-benzyloxycarbonyl $\gamma$-tert-butylester $\alpha$-L-glutamique, on obtient :

**EXEMPLE 16 : LA 1-(2,3,4-TRIMETHOXYBENZYL) 4-[N-BENZYLOXYCARBONYL $\gamma$-TERT-BUTYLESTER $\alpha$-L-GLUTAMYL] PIPERAZINE**

. la $N_\alpha$, $N_\delta$, $N_\omega$ -tribenzyloxycarbonyl L-arginine, on obtient :

**EXEMPLE 17 : LA 1-(2,3,4-TRIMETHOXYBENZYL) 4-[$N_\alpha$, $N_\delta$, $N_\omega$ -TRIBENZYLOXYCARBONYL L-ARGINYL] PIPERAZINE**

. la N-benzyloxycarbonyl O-tert-butyl L-tyrosine, on obtient :

**EXEMPLE 18 : LA 1-(2,3,4-TRIMETHOXYBENZYL) 4-[N-BENZYLOXYCARBONYL O-TERT-BUTYL L-TYROSYL] PIPERAZINE**

. la N-benzyloxycarbonyl O-tert-butyl L-thréonine on obtient :

**EXEMPLE 19 : LA 1-(2,3,4-TRIMETHOXYBENZYL) 4-[N-BENZYLOXYCARBONYL O-TERT-BUTYL L-THREONYL] PIPERAZINE**

. la N-benzyloxycarbonyl O-tert-butyl L-sérine, on obtient :

**EXEMPLE 20 : LA 1-(2,3,4-TRIMETHOXYBENZYL) 4-[N-BENZYLOXYCARBONYL O-TERT-BUTYL L-SERYL] PIPERAZINE**

. la $N_\alpha$-benzyloxycarbonyl $N_\epsilon$-tert-butyloxycarbonyl L-lysine, on obtient :

**EXEMPLE 21 : LA 1-(2,3,4-TRIMETHOXYBENZYL) 4-[$N_\alpha$-BENZYLOXYCARBONYL $N_\epsilon$-TERT-BUTYLOXYCARBONYL L-LYSYL] PIPERAZINE**

. la N-benzyloxycarbonyl O-benzyl D-tyrosine, on obtient :

**EXEMPLE 22 : LA 1-(2,3,4-TRIMETHOXYBENZYL) 4-[N-BENZYLOXYCARBONYL O-BENZYL D-TYROSYL] PIPERAZINE**

. la $N_\alpha$-benzyloxycarbonyl L-histidine, on obtient :

**EXEMPLE 23 : LA 1-(2,3,4-TRIMETHOXYBENZYL) 4-[$N_\alpha$-BENZYLOXYCARBONYL L-HISTIDYL] PIPERAZINE**

. l'acide N-benzyloxycarbonyl $\beta$-tert-butylester $\alpha$-L-aspartique, on obtient :

**EXEMPLE 24 : LA 1-(2,3,4-TRIMETHOXYBENZYL) 4-[N-BENZYLOXYCARBONYL $\beta$-TERTBUTYLESTER $\alpha$-L-ASPARTYL] PIPERAZINE**

. la $N_\alpha$-benzyloxycarbonyl L-asparagine, on obtient :

**EXEMPLE 25 : LA 1-(2,3,4-TRIMETHOXYBENZYL) 4-[$N_\alpha$-BENZOXYCARBONYL L-ASPARAGINYL] PIPERAZINE**

. l'acide 2-[1',3'-di(terbutyloxycarbonyl)guanidino]acétique, on obtient :

**EXEMPLE 26 : LA 1-(2,3,4-TRIMETHOXYBENZYL) 4-{2-[1',3'-DI(TERTBUTYLOXYCARBONYL)-GUANIDINO]ACETYL} PIPERAZINE**

. l'acide 2-[(3'-tertbutyloxycarbonyl 1'-méthyl)guanidino] acétique, on obtient :

**EXEMPLE 27 : LA 1-(2,3,4-TRIMETHOXYBENZYL) 4-{2-[(3'-TERBUTYLOXYCARBONYL 1'-METHYL)GUANIDINO]ACETYL} PIPERAZINE**

. l'acide 3-[1',3'-di(tertbutyloxycarbonyl)guanidino]propionique, on obtient :

**EXEMPLE 28 : LA 1-(2,3,4-TRIMETHOXYBENZYL) 4-{3-[1',3'-DI(TERTBUTYLOXYCARBONYL)-GUANIDINO]PROPIONYL} PIPERAZINE**

. l'acide 4-[1',3'-di(tertbutyloxycarbonyl)guanidino]butyrique, on obtient :

**EXEMPLE 29 : LA 1-(2,3,4-TRIMETHOXYBENZYL) 4-{4-[1',3'-DI(TERTBUTYLOXYCARBONYL)-GUANIDINO]BUTYRIL} PIPERAZINE**

. l'acide 2-benzyloxycarbonylamino 4,4-di(tertbutyloxycarbonyl)butyrique, on obtient :

**EXEMPLE 30 : LA 1-(2,3,4-TRIMETHOXYBENZYL) 4-[2-BENZYLOXYCARBONYLAMINO 4,4-DI-(TERTBUTYLOXYCARBONYL) BUTYRYL] PIPERAZINE**

. l'acide 2-benzyloxycarbonylamino 3,3-di(tertbutyloxycarbonyl)propionique, on obtient :

**EXEMPLE 31 : LA 1-(2,3,4-TRIMETHOXYBENZYL) 4-[2-BENZYLOXYCARBONYLAMINO 3,3-DI-(TERTBUTYLOXYCARBONYL) PROPIONYL] PIPERAZINE**

. la $N_\alpha$-benzyloxycarbonyl L-glutamine, on obtient :

**EXEMPLE 32 : LA 1-(2,3,4-TRIMETHOXYBENZYL) 4-[$N_\alpha$-BENZYLOXYCARBONYL L-GLUTAMINYL] PIPERAZINE**

. la N-benzyloxycarbonyl $\gamma$-trans-hydroxy L-proline, on obtient :

**EXEMPLE 33 : LA 1-(2,3,4-TRIMETHOXYBENZYL) 4-[N-BENZYLOXYCARBONYL $\gamma$-TRANS-HYDROXY L-PROLYL] PIPERAZINE**

. la N-benzyloxycarbonyl L-isoleucine, on obtient :

**EXEMPLE 34 : LA 1-(2,3,4-TRIMETHOXYBENZYL) 4-[N-BENZYLOXYCARBONYL L-ISOLEUCYL] PIPERAZINE**

. la $N_\alpha$-benzyloxycarbonyl $N_\omega$-nitro L-arginine, on obtient :

**EXEMPLE 35 : LA 1-(2,3,4-TRIMETHOXYBENZYL) 4-[$N_\alpha$-BENZYLOXYCARBONYL $N_\omega$-NITRO L-ARGINYL] PIPERAZINE**

. la N-benzyloxycarbonyl L-méthionine, on obtient :

**EXEMPLE 36 : LA 1-(2,3,4-TRIMETHOXYBENZYL) 4-[N-BENZYLOXYCARBONYL L-METHIONYL] PIPERAZINE**

. le $N_\alpha$-benzyloxycarbonyl L-tryptophane, on obtient :

**EXEMPLE 37 : LA 1-(2,3,4-TRIMETHOXYBENZYL) 4-[$N_\alpha$-BENZYLOXYCARBONYL L-TRYPTOPHYL] PIPERAZINE**

. l'acide N-benzyloxycarbonyl perhydroindol-2-yl(2S,3aS,7aS) carboxylique, on obtient :

**EXEMPLE 38 : LA 1-(2,3,4-TRIMETHOXYBENZYL) 4-{[N-BENZYLOXYCARBONYL PERHYDROINDOL-2-YL(2S,3aS,7aS)]CARBONYL} PIPERAZINE**

. la N,O-di(benzyloxycarbonyl) L-tyrosine, on obtient :

**EXEMPLE 39 : LA 1-(2,3,4-TRIMETHOXYBENZYL) 4-[N,O-DI(BENZYLOXYCARBONYL) L-TYROSYL] PIPERAZINE**

. la N,S-di(benzyloxycarbonyl) L-cystéine, on obtient :

**EXEMPLE 40 : LA 1-(2,3,4-TRIMETHOXYBENZYL) 4-[N,S-DI(BENZYLOXYCARBONYL) L-CYSTÉINYL] PIPERAZINE**

. la N-tert-butyloxycarbonyl S-benzyl L-cystéine, on obtient :

**EXEMPLE 41 : LA 1-(2,3,4-TRIMETHOXYBENZYL) 4-[N-TERTBUTYLOXYCARBONYL S-BENZYL L-CYSTÉINYL] PIPERAZINE**

. la N-pivaloyl S-benzyl L-cystéine, on obtient :

**EXEMPLE 42 : LA 1-(2,3,4-TRIMETHOXYBENZYL) 4-[N-PIVALOYL S-BENZYL L-CYSTÉINYL] PIPERAZINE**

. la N-acétyl S-benzyl L-cystéine, on obtient :

**EXEMPLE 43 : LA 1-(2,3,4-TRIMETHOXYBENZYL) 4-[N-ACETYL S-BENZYL L-CYSTÉINYL] PIPERAZINE**

. la N-palmitoyl S-benzyl L-cystéine, on obtient :

**EXEMPLE 44 : LA 1-(2,3,4-TRIMETHOXYBENZYL) 4-[N-PALMITOYL S-BENZYL L-CYSTEINYL] PIPERAZINE**

. la N-eicosanoyl S-benzyl L-cystéine, on obtient :

**EXEMPLE 45 : LA 1-(2,3,4-TRIMETHOXYBENZYL) 4-[N-EICOSANOYL S-BENZYL L-CYSTEINYL] PIPERAZINE**

**EXEMPLE 46 : LA 1-(2,5-DIMETHOXYBENZYL) 4-[S-BENZYL N-BENZYLOXYCARBONYL L-CYSTEINYL] PIPERAZINE**

En procédant comme dans l'exemple 1, mais en remplaçant au stade B la 1-(2,3,4-triméthoxybenzyl) pipérazine par la 1-(2,5-diméthoxybenzyl) pipérazine, on obtient le chlorhydrate de 1-(2,5-diméthoxybenzyl) 4-[S-benzyl N-benzyloxycarbonyl L-cystéinyl] pipérazine.

**EXEMPLES 47 A 49**

En procédant comme dans l'exemple 1, mais en remplaçant au stade B la 1-(2,3,4-triméthoxybenzyl) pipérazine par la 1-(2,5-diméthoxybenzyl) pipérazine, et en remplaçant au stade A la S-benzyl N-benzyloxycarbonyl L-cystéine

. par la N-benzyloxycarbonyl L-proline, on obtient :

**EXEMPLE 47 : LA 1-( 2,5-DIMETHOXYBENZYL) 4-[N-BENZYLOXYCARBONYL L-PROLYL] PIPERAZINE**

. par la S-éthylthio N-benzyloxycarbonyl L-cystéine on obtient :

**EXEMPLE 48 : LA 1-(2,5-DIMETHOXYBENZYL) 4-[S-ETHYLTHIO N-BENZYLOXYCARBONYL L-CYSTEINYL] PIPERAZINE**

. par la N-benzyloxycarbonyl L-alanine, on obtient :

**EXEMPLE 49 : LA 1-(2,5-DIMETHOXYBENZYL) 4-[N-BENZYLOXYCARBONYL L-ALANYL] PIPERAZINE**

**EXEMPLE 50 : LA 1-( 3-METHOXYBENZYL) 4-[S-(BENZYL) N-(BENZYLOXYCARBONYL) L-CYSTEINYL]-PIPERAZINE**

En procédant comme dans l'exemple 1, mais en remplaçant au stade B la 1-(2,3,4-triméthoxybenzyl) pipérazine par la 1-(3-méthoxybenzyl) pipérazine, on obtient le chlorhydrate de 1-(3-méthoxybenzyl) 4-[S-benzyl N-benzyloxycarbonyl L-cystéinyl] pipérazine.

**EXEMPLES 51 A 63**

En procédant comme dans l'exemple 3, mais en remplaçant la 1-(2,3,4-triméthoxybenzyl) 4-[N-benzyloxycarbonyl L-prolyl] pipérazine par :

. la 1-(2,3,4-triméthoxybenzyl) 4-[N-benzyloxycarbonyl L-valyl] pipérazine, on obtient :
**EXEMPLE 51 : LA 1-(2,3,4-TRIMETHOXYBENZYL) 4-(L-VALYL) PIPERAZINE**

. la 1-(2,3,4-triméthoxybenzyl) 4-[N-benzyloxycarbonyl 4-aminobutyryl] pipérazine, on obtient :
**EXEMPLE 52 : LA 1-(2,3,4-TRIMETHOXYBENZYL) 4-(4-AMINOBUTYRYL) PIPERAZINE**

. la 1-(2,3,4-triméthoxybenzyl) 4-[N-benzyloxycarbonyl $\gamma$-tert-butylester $\alpha$-L-glutamyl] pipérazine, on obtient :
**EXEMPLE 53 : LA 1-(2,3,4-TRIMETHOXYBENZYL) 4-[$\gamma$-TERT-BUTYLESTER $\alpha$-L-GLUTAMYL] PIPERAZINE**

. la 1-(2,3,4-triméthoxybenzyl) 4-[N-benzyloxycarbonyl O-tert-butyl L-tyrosyl] pipérazine, on obtient :
**EXEMPLE 54 : LA 1-(2,3,4-TRIMETHOXYBENZYL) 4-[O-TERT-BUTYL L-TYROSYL] PIPERAZINE**

. la 1-(2,3,4-triméthoxybenzyl) 4-[N-benzyloxycarbonyl O-tert-butyl L-séryl] pipérazine, on obtient :
**EXEMPLE 55 : LA 1-(2,3,4-TRIMETHOXYBENZYL) 4-[O-TERT-BUTYL L-SERYL] PIPERAZINE**

. la 1-(2,3,4-triméthoxybenzyl) 4-[$N_\alpha$-benzyloxycarbonyl $N_\epsilon$-tert-butyloxycarbonyl L-lysyl] pipérazine, on obtient :
**EXEMPLE 56 : LA 1-(2,3,4-TRIMETHOXYBENZYL) 4-[$N_\epsilon$-TERT-BUTYLOXYCARBONYL L-LYSYL] PIPERAZINE**

. la 1-(2,3,4-triméthoxybenzyl) 4-[$N_\alpha$-benzyloxycarbonyl L-histidyl] pipérazine, on obtient :
**EXEMPLE 57 : LA 1-(2,3,4-TRIMETHOXYBENZYL) 4-(L-HISTIDYL) PIPERAZINE**

. la 1-(2,3,4-triméthoxybenzyl) 4-[N-benzyloxycarbonyl L-glutaminyl] pipérazine, on obtient :
**EXEMPLE 58 : LA 1-(2,3,4-TRIMETHOXYBENZYL) 4-(L-GLUTAMINYL) PIPERAZINE**

. la 1-(2,3,4-triméthoxybenzyl) 4-{[N-benzyloxycarbonyl perhydroindol-2-yl(2S,3aS,7aS)]carbonyl} pipérazine, on obtient :
**EXEMPLE 59 : LA 1-(2,3,4-TRIMETHOXYBENZYL) 4-[(PERHYDROINDOL-2-YL(2S,3aS,7aS)-CARBONYL] PIPERAZINE**

. la 1-(2,5-diméthoxybenzyl) 4-[S-benzyl N-benzyloxycarbonyl L-cystéinyl] pipérazine, on obtient :
**EXEMPLE 60 : LA 1-(2,5-DIMETHOXYBENZYL) 4-[S-BENZYL L-CYSTEINYL] PIPERAZINE**

. la 1-(2,5-diméthoxybenzyl) 4-[N-benzyloxycarbonyl L-alanyl] pipérazine, on obtient :
**EXEMPLE 61 : LA 1-(2,5-DIMETHOXYBENZYL) 4-(L-ALANYL) PIPERAZINE**

. la 1-(3-méthoxybenzyl) 4-[S-benzyl N-benzyloxycarbonyl L-cystéinyl] pipérazine, on obtient :
**EXEMPLE 62 : LA 1-(3-METHOXYBENZYL) 4-[S-BENZYL L-CYSTEINYL] PIPERAZINE**

. la 1-(2,3,4-triméthoxybenzyl) 4-[$N_\alpha$, $N_\delta$, $N_\omega$-tribenzyloxycarbonyl L-arginyl] pipérazine, on obtient :
**EXEMPLE 63 : LA 1-(2,3,4-TRIMETHOXYBENZYL) 4-(L-ARGINYL)PIPERAZINE**

**EXEMPLE 64 : LA 1-(2,3,4-TRIMETHOXYBENZYL) 4-(L-LYSYL) PIPERAZINE**

20 $cm^3$ d'acide trifluoroacétique sont ajoutés à une solution de 0,9 grammes de 1-(2,3,4-triméthoxybenzyl) 4-[$N_\epsilon$-tert-butyloxycarbonyl L-lysyl] pipérazine dans 20 $cm^3$ de chlorure de méthylène.
On laisse sous agitation durant 20 mn à 20°C.Après évaporation du chlorure de méthylène, et élimination de l'acide trifluoroacétique on obtient la 1-(2,3,4-triméthoxybenzyl) 4-(L-lysyl) pipérazine sous forme d'huile.

Cette huile peut-être reprise dans de l'eau pour donner après acidification avec de l'acide chlorhydrique 1N et isolement par lyophilisation le chlorhydrate de 1-(2,3,4-triméthoxybenzyl) 4-(L-lysyl) pipérazine.

**EXEMPLES 65 A 69**

En procédant comme dans l'exemple 65, mais en remplaçant la 1-(2,3,4-triméthoxybenzyl) 4-[$N_\epsilon$-tertbutyloxycarbonyl L-lysyl] pipérazine par :

. la 1-(2,3,4-triméthoxybenzyl) 4-[$N_\alpha$-benzyloxycarbonyl N-tert butyloxycarbonyl L-lysyl] pipérazine, on obtient :

**EXEMPLE 65 : LA 1-(2,3,4-TRIMETHOXYBENZYL) 4-($N_\alpha$-BENZYLOXYCARBONYL L-LYSYL) PIPERAZINE**

. la 1-(2,3,4-triméthoxybenzyl) 4-{2-[1',3'-di(tert butyloxycarbonyl)guanidino]acétyl} pipérazine, on obtient :

**EXEMPLE 66 : LA 1-(2,3,4-TRIMETHOXYBENZYL) 4-(2-GUANIDINO ACETYL) PIPERAZINE**

. la 1-(2,3,4-triméthoxybenzyl) 4-[2-(3'-tertbutyloxycarbonyl 1'-méthylguanidino)acétyl] pipérazine, on obtient :

**EXEMPLE 67 : LA 1-(2,3,4-TRIMETHOXYBENZYL) 4-(2-(1'-METHYLGUANIDINO)ACETYL] PIPERAZINE**

. la 1-(2,3,4-triméthoxybenzyl) 4-{3-[1',3'-di (tertbutyloxycarbonyl)guanidino]propionyl} pipérazine, on obtient :

**EXEMPLE 68 : LA 1-(2,3,4-TRIMETHOXYBENZYL) 4-(3-GUANIDINO PROPIONYL) PIPERAZINE**

. la 1-(2,3,4-triméthoxybenzyl) 4-{4-[1',3'-di (tertbutyloxycarbonyl)guanidino]butyryl} pipérazine, on obtient :

**EXEMPLE 69 : LA 1-(2,3,4-TRIMETHOXYBENZYL) 4-(4-GUANIDINO BUTYRYL) PIPERAZINE**

**EXEMPLE 70 : LA 1-(2,3,4-TRIMETHOXYBENZYL) 4-(2-AMINO 4,4-DICARBOXYBUTYRYL) PIPERAZINE**

En procédant comme dans l'exemple 3 mais en remplaçant la 1-(2,3,4-triméthoxybenzyl) 4-[N-benzyloxycarbonyl L-propyl] pipérazine par la 1-(2,3,4-triméthoxybenzyl) 4-[2-benzyloxycarbonylamino 4,4-di(tertbutyloxycarbonyl)butyryl] pipérazine, puis en réalisant une étape d'hydrolyse acide douce on obtient la 1-(2,3,4-triméthoxybenzyl) 4-[2-amino 4,4-dicarboxy butyryl) pipérazine.

**EXEMPLE 71 : LA 1-(2,3,4-TRIMETHOXYBENZYL) 4-(2-AMINO 3,3-DICARBOXYPROPIONYL) PIPERAZINE**

En procédant comme dans l'exemple 71, mais en utilisant la 1-(2,3,4-triméthoxybenzyl) 4-[2-benzyloxycarbonylamino 3,3-di(terbutyloxycarbonyl) propionyl] pipérazine à la place de 1-(2,3,4-triméthoxybenzyl) 4-[2-benzyloxycarbonylamino 4,4-di(tertbutyloxycarbonyl) butyryl] pipérazine, on obtient la 1-(2,3,4-triméthoxybenzyl) 4-(2-amino 3,3-dicarboxypropionyl) pipérazine.

**EXEMPLE 72 : LA 1-(2,3,4-TRIMETHOXYBENZYL) 4-{2-{{4-[1', 3'-DI(TERTBUTYLOXYCARBONYL)-GUANIDINO]BUTYRYL}AMINO}ACETYL} PIPERAZINE**

En procédant comme dans l'exemple 1 mais en remplaçant au stade A la S-benzyl N-benzyloxycarbonyl L-cystéine par l'acide 4-[1',3'-di(tertbutyloxycarbonyl)guanidino]butyrique et au stade B la 1-(2,3,4-triméthoxybenzyl)pipérazine par la 1-(2,3,4-triméthoxybenzyl) 4-glycylpipérazine obtenue à l'exemple 13, on obtient la 1-(2,3,4-triméthoxybenzyl) 4-{2-{ {4-[1',3'-di(tertbutyloxycarbonyl)guanidino] butyryl}amino}acétyl} pipérazine

**ETUDE PHARMACOLOGIOUE DES COMPOSES DE LA PRESENTE INVENTION**

**EXEMPLE A : Recherche de l'activité anti-hypoxique in vitro**

<u>Principe</u> :

Les astrocytes en culture constituent un modèle de choix pour la recherche d'une activité cytoprotectrice face à une situation d'hypoxie.

En effet, la première réaction cellulaire visible à toute agression cérébrale est un gonflement astrocytaire, alors même que les neurones, les oligodendrocytes et les cellules endothéliales ont encore un profil morphologique normal.

En outre, il a été montré récemment que l'astrocyte avait un rôle majeur au niveau cérébral, notamment dans l'élaboration des acides aminés neurotransmetteurs et dans la préservation de l'équilibre ionique extracellulaire (Rothman, S.M. et al. Ann. Neurol. (1986) ; 19 : 105-111).

La demanderesse a donc testé l'effet des composés de l'invention sur la protection cellulaire d'astrocytes en culture placés en situation d'hypoxie :

- en mesurant la consommation en oxygène de ces astrocytes, ce qui permet un suivi de l'activité respiratoire,
- en analysant certains marqueurs enzymatiques, qui permettent de mesurer l'intégrité métabolique et membranaire de ces astrocytes.

Méthodologie :

Les astrocytes de rats en culture primaire sont préparés à partir de cortex provenant de cerveaux de rats nouveau-nés.

Le traitement hypoxique consiste en une exposition des cellules, en atmosphère humide, à un mélange gazeux constitué de 95 % N2 et 5 % $CO_2$, à 37°C pendant 15 heures.

Les composés à tester sont ajoutés au milieu de culture 12 heures avant l'hypoxie. Un second ajout est réalisé à la fin de la période hypoxique.

2 heures après la fin de l'hypoxie, on mesure la consommation cellulaire d'oxygène ainsi que l'activité enzymatique lactate déshydrogénase (LDH) intra-cellulaire et extra-cellulaire et le rapport des différentes isoenzymes de la LDH (isoenzyme Heart/isoenzyme Muscle ou H/M).

a) Consommation d'oxygène : On détermine la consommation d'oxygène des cellules en milieu salin isotonique par mesure polarimétrique dans un oxygraphe GILSON, muni d'une électrode de Clark à oxygène.

b) Activité LDH intra-cellulaire : Elle est déterminée par mesure spectrophotométrique à 340 nm sur un extrait cellulaire obtenu après désintégration des cellules aux ultrasons.

c) Activité LDH extra-cellulaire : Elle est déterminée par mesure spectrophotométrique à 340 nm sur le milieu de culture.

d) Rapport H/M : Les différentes formes isoenzymatiques intracellulaires de la LDH sont estimées par électrophorèse en gel de polyacrylamide à 7 % et identification spécifique des bandes isoenzymatiques.

Résultats :

Les composés de l'invention permettent une amélioration de l'activité respiratoire (consommation d'oxygène), après hypoxie des cellules, largement supérieure à celle obtenue après traitement avec de la trimétazidine.

En outre, l'analyse des différents marqueurs enzymatiques montre que les composés de l'invention présentent un effet protecteur remarquable de l'intégralité métabolique (activité LDH intra-cellulaire et rapport des isoenzymes : H/M) et de l'intégrité membranaire (activité LDH extra-cellulaire).

A titre d'exemple, la chlorhydrate de 1-(2,3,4-triméthoxybenzyl) 4-[S-benzyl N-benzyloxycarbonyl L-cystéinyl] pipérazine provoque une amélioration de l'activité respiratoire de 34 % par rapport aux cellules non traitées.

Le chlorhydrate de 1-(2,3,4-triméthoxybenzyl) 4-[S-benzyl N-benzyloxycarbonyl L-cystéinyl] pipérazine augmente également de plus de 20 % la valeur du rapport H/M, une augmentation de la valeur du rapport étant le signe d'un bon fonctionnement métabolique, alors qu'on observe une baisse de 17 % de la valeur de ce rapport dans les cellules non traitées.

**Exemple B : Recherche d'une activité anti-ischémiante in vivo**

Principe :

Certaines gerbilles (40 à 60 % des cas), dites sensibles, présentent une anomalie du cercle de Willis (Levine et al. Exp. Neurol. 1966 ; 16 : 255 - 262).

Grâce à cette anomalie, l'occlusion d'une carotide chez la gerbille permet de reproduire, contrairement aux autres espèces animales, la pathologie de l'ischémie cérébrale humaine.

La demanderesse a donc présentement testé l'influence des composés de l'invention sur la survie des gerbilles ayant subi une ischémie cérébrale par ligature de la carotide gauche.

Méthodologie :

On anesthésie les gerbilles "sensibles" au Kétalar® à la dose de 60 mg/kg par voie intapéritonéale. 30 minutes avant la ligature de la carotide gauche, on administre, par la voie intrapéritonéale, différentes concentrations des composés à tester en solution dans le polyéthylène glycol à 3 %.

On analyse le comportement des animaux à différents temps et les signes observés sont traduits en score selon l'indice de Mac Graw modifié (Mac Graw, CP et al. ; Stroke (1976) ; 7 : 485).
L'étude statistique est faite selon le test U de Mann Whitney.

Résultats :

Les composés de l'invention présentent une activité protectrice anti-ischémiante très importante et bien supérieure à celle de la trimétazidine.

A titre d'exemple, le pourcentage de survie des animaux après ligature de la carotide gauche dépasse les 70 %, au temps 96 h, sous l'action du chlorhydrate de 1-(2,3,4-triméthoxybenzyl) 4-[S-benzyl N-benzyloxycarbonyl L-cystéinyl] pypérazine, alors que tous les animaux sont morts dans les expériences contrôle.

**Exemple C : Recherche d'une activité anti-hypoxique in vivo**

Principe :

les animaux (souris) sont placés dans une atmosphère pauvre en oxygène, ce qui provoque l'apparition de suffocations (ou "gasps").

Les composés possèdant des propriétés anti-hypoxiques provoquent un retard dans l'apparition de ces suffocations.

La demanderesse a présentement testé les composés de l'invention dans ce test.

Méthodologie :

Les souris mâles (Swiss CD1) pesant 25 - 30 g sont stabulées durant 1 semaine avant toute expérience dans les conditions usuelles d'animalerie (20-22 °C, 55 % d'humidité, cycle lumière/obscurité 12/12, nourriture industrielle et eau à volonté).

Les souris sont placées dans une boite (7 × 5 × 5 cm) dans laquelle on crée une atmosphère pauvre en oxygène par le passage d'un courant d'air (96 % $N_2$, 4 % $O_2$, 12 l/mn).

Le délai d'apparition des premières suffocations ou ("gasps") est mesuré.

Les souris reçoivent une dose des composés à tester par voie intrapéritonéale 30 mn avant la réalisation de l'hypoxie.

La vincamine (14,15-dihydro 14-hydroxyeburnamenine 14-carboxylate de méthyle) est utilisée comme drogue de référence.

Résultats :

Les résultats sont présentés dans le tableau 1 pour le composé de l'invention qui est le chlorhydrate de 1-(2,3,4-triméthoxybenzyl) 4-[S-benzyl N-benzyloxycarbonyl L-cystéinyl] pipérazine.

| TRAITEMENT | DOSE (mg/kg) | DELAI D'APPARITION DES PREMIERES SUFFOCATIONS OU "GASPS" (en secondes) |
|---|---|---|
| TEMOIN | - | 30 ± 7 |
| VINCAMINE | 2,5 | 48 ± 8 (non significatif) |
| VINCAMINE | 20 | 167 ± 12 (significatif) |
| CHLORHYDRATE DE 1-(2,3,4-TRIMETHOXYBENZYL) 4-[S-BENZYL N-BENZYLOXYCARBONYL L-CYSTEINYL] PIPERAZINE | 2,5 | 70 ± 13 (significatif) |

Le résultats montrent que le chlorhydrate de 1-(2,3,4-triméthoxybenzyl) 4-[S-benzyl N-benzyloxycarbonyl L-cystéinyl] pipérazine est actif comme antihypoxique dès la dose de 2,5 mg/kg alors que le composé de référence, à la même dose, ne provoque aucune différence significative.

**Revendications**

**Revendications pour les Etats contractants suivants : AT, BE, CH, LI, DE, DK, FR, GB, IT, LU, NL, PT, SE, IE**

**1.** Composés de formule générale (I) :

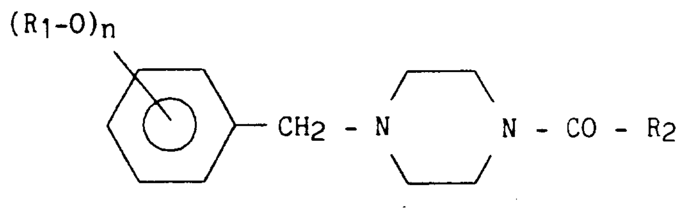

(I)

dans laquelle :

- n, nombre entier, peut prendre les valeurs 1, 2, ou 3,
- $R_1$ est un alkyle linéaire ou ramifié contenant de 1 à 4 atomes de carbone,
- $R_2$ représente :
  un groupement

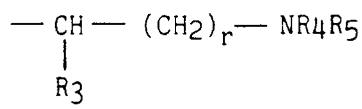

dans lequel :

- r nombre entier peut prendre les valeurs 0, 1, 2,
- $R_3$ représente :
  - a) un atome d'hydrogène,
  - b) un alkyle linéaire ou ramifié de 1 à 6 atomes de carbone éventuellement substitué par
    * un ou deux groupements choisis parmi :
      - COOH, $-CO-R_6$, $-CO-O-R_6$ avec $R_6$ étant un groupement choisi parmi : alkyle linéaire ou ramifié saturé, insaturé, ou polyinsaturé de 1 à 12 atomes de carbone, et $-(CH_2)_m$-aryle éventuellement substitué où m, nombre entier, peut prendre les valeurs 0, 1, 2, ou 3,
    * ou par un groupement choisi parmi :
      i) -OH, $-O-R_6$, $-O-CO-R_6$, ou $-O-CO-O-R_6$ avec $R_6$ tel que défini précédemment,
      ii) $-NR_7R_8$ avec R7 et $R_8$, identiques ou différents, représentant chacun indépendamment l'un de l'autre un atome d'hydrogène, un alkyle linéaire ou ramifié de 1 à 6 atomes de carbone $-CO-R_6$ ou $-CO-O-R_6$ avec $R_6$ ayant la même signification que précédemment, et $-(CH_2)_m$-aryle éventuellement substitué, où m nombre entier peut prendre les valeurs 0, 1, 2, ou 3,
      iii) $-CO-NR_9R_{10}$ avec $R_9$ et $R_{10}$, identiques ou différents, ayant la même définition que $R_6$ avec $R_6$ tel que défini précedemment, ou pouvant également représenter un atome d'hydrogène, ou $R_9$ et $R_{10}$ forment ensemble, avec l'atome d'azote qui les porte, un système cyclique de 5 à 7 sommets pouvant comprendre éventuellement un second hétéroatome choisi parmi oxygène, azote, et soufre,
      iv) -SH, ou -SeH,
      v) un groupement $-S-R_6$, $-Se-R_6$, $-S-CO-O-R_6$, $-S-S-R_6$ ou $-Se-Se-R_6$ avec $R_6$ tel que défini précédemment,
      vi) un radical guanidino non substitué ou substitué par 1 à 2 groupements choisi parmi nitro et $-CO-O-R_6$ avec $R_6$ tel que défini précédemment,
      vii) un groupement indole ou imidazole éventuellement substitué,
  . c) un groupement $-(CH_2)_m$-phényle éventuellement substitué où m, nombre entier, peut prendre les valeurs 0, 1, 2, ou 3,
  étant entendu que $R_3$ ne peut pas représenter un groupement isobutyle,
- $R_4$ représente un atome d'hydrogène, un alkyle linéaire ou ramifié de 1 à 6 atomes de carbone, un groupement $-(CH_2)_p$-aryle éventuellement substitué où p, nombre entier peut

prendre les valeurs 0, 1, 2, ou 3, ou un groupement amidino non substitué ou substitué par un groupement nitro ou -CO-O-$R_6$ avec $R_6$ tel que défini précédemment,

ou $R_4$ forme avec $R_3$ et les atomes auxquels ils sont liés un système mono ou bicyclique choisi parmi : pyrrolidine, pipéridine, perhydroindole, indoline, 2-aza bicyclo[2, 2, 2]octane, et 2-aza bicyclo[3, 3, 0]octane, ces systèmes cycliques pouvant être éventuellement substitués par un ou plusieurs groupements choisis parmi :

hydroxy, oxo, alkyle linéaire ou ramifié de 1 à 6 atomes de carbone, alcoxy linéaire ou ramifié de 1 à 6 atomes de carbone,

avec la réserve que $R_3$ et $R_4$ ne peuvent pas former ensemble, avec les atomes auxquels ils sont liés, une pyrrolidine substituée par un groupement oxo en a de l'azote,

- $R_5$ représente :
  - un atome d'hydrogène,
  - un groupement $-(CH_2)_q$-aryle éventuellement substitué où q, nombre entier, peut prendre les valeurs 0, 1, 2, ou 3,
  - une chaine alkyle de 1 à 20 atomes de carbone, linéaire ou ramifiée, saturée, insaturée ou polyinsaturée, éventuellement interrompue par un ou plusieurs atomes d'oxygène, de soufre, ou d'azote, et éventuellement substituée par un ou plusieurs radicaux hydroxy, amino, alkylamino linéaire ou ramifié de 1 à 6 atomes de carbone, ou alcoxy linéaire ou ramifié de 1 à 6 atomes de carbone,
  - un groupement -CO-$R_{11}$ ou -CO-O-$R_{11}$ où $R_{11}$ représente :
    . une chaine alkyle de 1 à 20 atomes de carbone, linéaire ou ramifiée, saturée, insaturée ou polyinsaturée, éventuellement interrompue par un ou plusieurs atomes d'oxygène, de soufre, ou d'azote, et éventuellement substituée par un ou plusieurs radicaux hydroxy, amino, alkylamino linéaire ou ramifié de 1 à 6 atomes de carbone, alcoxy linéaire ou ramifié de 1 à 6 atomes de carbone, guanidino, ou guanidino substitué par 1 à 2 groupements choisis parmi nitro et -CO-O-$R_6$ avec $R_6$ tel que défini précédemment,
    . un groupement $-(CH_2)_q$-aryle éventuellement substitué où q, nombre entier, peut prendre les valeurs 0, 1, 2, ou 3,

étant entendu que, sauf précisions contraires, :

- le terme "aryle" désigne les radicaux phényle et naphtyle,
- le terme "substitué" associé aux expressions "$(CH_2)_m$-phényle", "$-(CH_2)_m$-aryle", "$-(CH_2)_p$-aryle", "$-(CH_2)_q$-aryle", "imidazole", et "indole" signifie que ces radicaux peuvent être substitués sur le cycle par un ou plusieurs groupements choisis parmi : hydroxy, halogène, nitro, trifluorométhyle, alkyle linéaire ou ramifié de 1 à 6 atomes de carbone, alcoxy linéaire ou ramifié de 1 à 6 atomes de carbone, $-(CH_2)_t$-phényle, -O-$(CH_2)_t$-phényle, et O-CO-O-$(CH_2)_t$-phényl, où t, nombre entier, peut prendre les valeurs 0, 1, 2, ou 3,
- et leurs isomères, diastéréoisomères, épimères, isolés ou sous forme de mélange ainsi que, le cas échéant, leurs sels d'addition à un acide ou à une base pharmaceutiquement acceptable.

**2.** Composés selon la revendication 1 pour lesquels n est égal à 3 et $R_1$ représente un méthyle, les trois groupements méthoxy ainsi définis étant portés en positions 2, 3, et 4 par le noyau aromatique de la benzylpipérazine, ce qui correspond aux composés de formule générale (I $\alpha$)

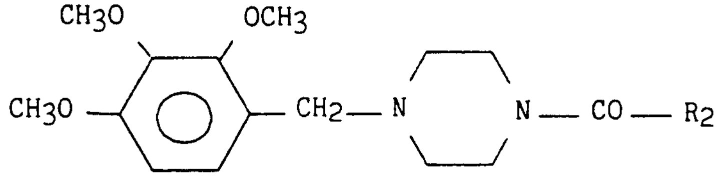

(I α)

dans laquelle $R_2$ est tel que défini dans la revendication 1 :

- leurs isomères, diastéréoisomères, épimères isolés ou sous forme de mélange,
- et leurs sels d'addition à un acide ou à une base pharmaceutiquement acceptable.

3. Composés selon la revendication 1 pour lesquels $R_2$ représente un groupement

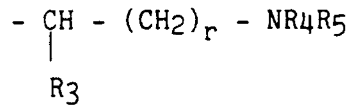

tel que défini dans la revendication 1, dans lequel $R_3$ représente spécifiquement un alkyle linéaire ou ramifié de 1 à 6 atomes de carbone substitué par un groupement -SH ou -S-$R_6$ avec $R_6$ tel que défini dans la revendication 1,
- leurs isomères, diastéréoisomères, épimères, isolés ou sous forme de mélange
- et leurs sels d'addition à un acide ou à une base pharmaceutiquement acceptable.

4. Composés selon la revendication 1 pour lesquels
- n est égal à 3,
- $R_1$ est un groupement méthyl, les trois groupements méthoxy ainsi définis étant portés en position 2, 3 et 4 par le noyau aromatique de la benzylpipérazine,
- $R_2$ est un groupement

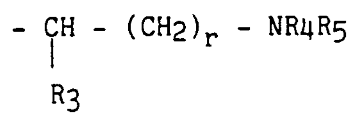

  tel que défini dans la revendication 1 pour lequel $R_3$ représente un alkyl linéaire ou ramifié de 1 à 6 atomes de carbone substitué par un groupement -SH ou -S-$R_6$ avec $R_6$ tel que défini dans la revendication 1,
- leurs isomères, diastéréoisomères, épimères isolés ou sous forme de mélange,
- et leurs sels d'addition à un acide ou à une base pharmaceutiquement acceptable.

5. Composés selon la revendication 1 pour lesquels $R_2$ représente un groupement

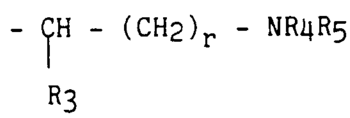

tel que défini dans la revendication 1, dans lequel $R_3$ et $R_4$ forment ensemble, avec les atomes auxquels ils sont liés un système mono ou bicyclique choisi parmi : pyrrolidine, pipéridine, perhydroindole, indoline, 2-aza bicyclo[2, 2, 2] octane, et 2-aza bicyclo(3, 3, 0] octane, ces systèmes cycliques pouvant être éventuellement substitués par un ou plusieurs groupements choisis parmi :

hydroxy, oxo, alkyle linéaire ou ramifié de 1 à 6 atomes de carbone, alcoxy linéaire ou ramifié de 1 à 6 atomes de carbone,

avec la réserve que $R_3$ et $R_4$ ne peuvent pas former ensemble, avec les atomes auxquels ils sont liés, une pyrrolidine substituée par un groupement oxo en $\alpha$ de l'azote,
- leurs isomères, diastéréoisomères, épimères, isolés ou sous forme de mélange,
- et leurs sels d'addition à un acide ou à une base pharmaceutiquement acceptable.

6. Composés selon la revendication 1 pour lesquels $R_3$ représente un alkyle linéaire ou ramifié de 1 à 6 atomes de carbone éventuellement substitué par un radical guanidino non substitué ou substitué par 1 à 2 groupements choisis parmi nitro et -CO-O-$R_6$ avec $R_6$ tel que défini dans la revendication 1,
- leurs isomères, diastéréoisomères, épimères, isolés ou sous forme de mélange,
- et leurs sels d'addition à un acide ou à une base pharmaceutiquement acceptable.

7. Le composé selon la revendication 1 qui est la 1-(2,3,4-triméthoxybenzyl) 4-[S-benzyl N-benzyloxycarbonyl L-cystéinyl] pipérazine, ses isomères optiques, isolés ou sous forme de mélange, et ses sels d'addition à un acide pharmaceutiquement acceptable.

**8.** Le composé selon la revendication 1 qui est la 1-(2,3,4-triméthoxybenzyl) 4-[N-benzyloxycarbonyl L-alanyl] pipérazine, ses isomères optiques, isolés ou sous forme de mélange, et ses sels d'addition à un acide pharmaceutiquement acceptable.

**9.** Le composé selon la revendication 1 qui est la 1-(2,3,4-triméthoxybenzyl) 4-glycyl pipérazine et ses sels d'addition à un acide pharmaceutiquement acceptable.

**10.** Le composé selon la revendication 1 qui est la 1-(2,3,4-triméthoxybenzyl) 4-[N-benzyloxycarbonyl L-prolyl] pipérazine, ses isomères optiques, isolés ou sous forme de mélange, et ses sels d'addition à un acide pharmaceutiquement acceptable.

**11.** Le composé selon la revendication 1 qui est la 1-(2,3,4-triméthoxybenzyl) 4-[S-benzyl L-cystéinyl] pipérazine, ses isomères optiques, isolés ou sous forme de mélange, et ses sels d'addition à un acide pharmaceutiquement acceptable.

**12.** Le composé selon la revendication 1 qui est la 1-(2,3,4-triméthoxybenzyl) 4-(L-arginyl) pipérazine, ses isomères optiques, isolés ou sous forme de mélange, et ses sels d'addition à un acide pharmaceutiquement acceptable.

**13.** Le composé selon la revendication 1 qui est la 1-(2,3,4-triméthoxybenzyl) 4-[$N_\alpha$, $N_\delta$, $N_\omega$-tribenzyloxycarbonyl L-arginyl) pipérazine, ses isomères optiques, isolés ou sous forme de mélange, et ses sels d'addition à un acide pharmaceutiquement acceptable.

**14.** Le composé selon la revendication 1 qui est la 1-(2,3,4-triméthoxybenzyl) 4-(2-amino 4,4-dicarboxybutyryl] pipérazine, ses isomères optiques, isolés ou sous forme de mélange, et ses sels d'addition à un acide pharmaceutiquement acceptable.

**15.** Procédé de préparation des composés de formule (I) selon la revendication 1, caractérisé en ce que l'on condense, en utilisant les techniques de couplage passant par la formation d'un ester activé (hydroxy succinimide, hydroxybenzotriazole, en présence de dicyclohexyl carbodiimide), un composé de formule (II) :

$HOOC - R'_2$ (II)

dans laquelle $R'_2$ a la même signification que $R_2$ dans la revendication 1 avec la réserve que lorsque $R'_2$ représente un groupement

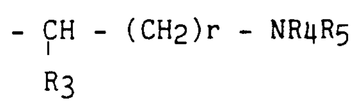

tel que défini dans la revendication 1 et que $R_4$ représente un atome d'hydrogène, alors $R_5$ représente un groupement $-CO-R_{11}$ ou $-CO-O-R_{11}$ avec $R_{11}$ ayant la même signification que dans la revendication 1,
avec une (alcoxybenzyl) pipérazine de formule (III) :

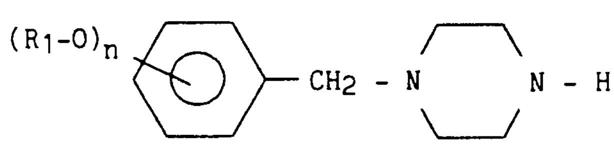

(III)

dans laquelle $R_1$ et n sont tels que définis dans la formule (I) afin d'obtenir un composé de formule (I/a) :

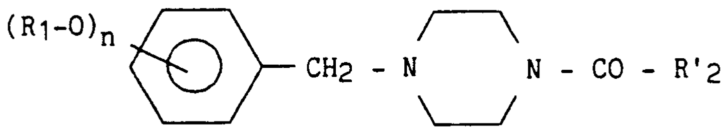

(I/a)

dans laquelle $R_1$, $R'_2$ et n sont tels que définis précédemment,
composés de formule (I/a) qui sont, dans le cas où $R_5$ représente un groupement $-CO-O-R_{11}$, éventuellement soumis si on le désire, aux réactions de déprotection des fonctions aminées en synthèse peptidique (traitement acide ou hydrogénation catalytique suivant la nature de $R_{11}$) afin d'accéder aux composés de formule (I) selon la revendication 1 pour lesquels $R_5$ représente un hydrogène,
les composés de formule (I) selon la revendication 1 pour lesquels $R_5$ représente un atome d'hydrogène pouvant ensuite, le cas échéant, être mis en réaction avec un composé de formule (IV) :

$HOOC-R_{11}$ (IV)

dans laquelle $R_{11}$ est tel que défini précédemment, afin d'obtenir un composé de formule (I/b) :

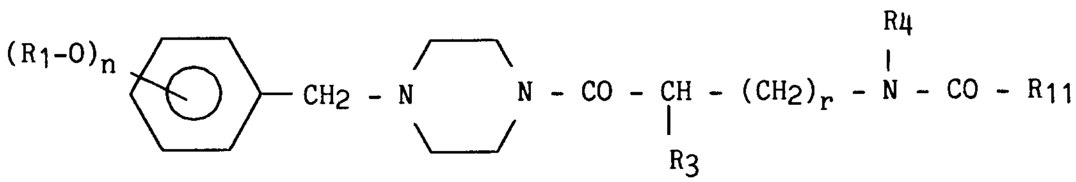

(I/b)

dans laquelle $R_1$, $R_3$, $R_4$, $R_{11}$ et n sont tels que définis précédemment,
cas particulier des composés de formule (I) selon la revendication 1 dans lesquels $R_2$ représente un groupement de formule :

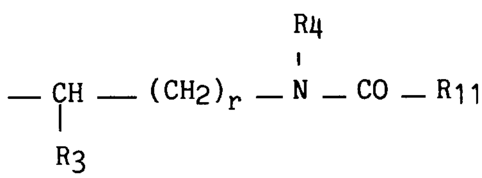

les composés de formule (I) selon la revendication 1 pouvant, si nécessaire, être purifiés, séparés, le cas échéant, en leurs différents isomères optiques, et pouvant également être salifiés, soit avec un acide pharmaceutiquement acceptable, soit, si cela est possible et si on le désire avec une base pharmaceutiquement acceptable.

**16.** Composition pharmaceutique contenant comme principe actif au moins un composé selon l'une des revendications 1 à 14 en combinaison avec un ou plusieurs excipients ou véhicules pharmaceutiquement acceptables.

**17.** Composition pharmaceutique selon la revendication 16, utilisable pour le traitement et la prévention de l'ischémie cérébrale, de l'hypoxie vasculaire cérébrale, de l'anoxie vasculaire cérébrale et des oedèmes cérébraux, pour le traitement des accidents vasculaires cérébraux, des traumatismes crâniens, des encéphalopathies, des maladie neuro-dégénératives et des troubles de la sénescence, ainsi que pour le traitement et la prévention de l'ischémie de type périphérique, en cardiologie : l'ischémie myocardique et l'ischémie coronarienne et leurs différentes expressions cliniques : angine de poitrine, infarctus du myocarde, troubles du rythme, spasme vasculaire, insuffisance cardiaque, ainsi qu'en ophtamologie et en oto-rhino-laryngologie lors d'atteintes vasculaires chorio-rétiniennes, de vertiges d'origine vasculaire, de vertiges de Menière ou d'acouphènes.

**Revendications pour les Etats contractants suivants : ES, GR**

**1.** Procédé de préparation de composés de formule générale (I) :

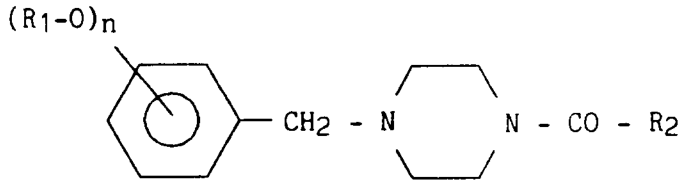

(I)

dans laquelle :

- n, nombre entier, peut prendre les valeurs 1, 2, ou 3,
- $R_1$ est un alkyle linéaire ou ramifié contenant de 1 à 4 atomes de carbone,
- $R_2$ représente :
  un groupement

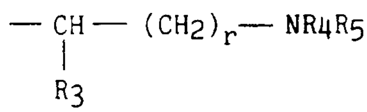

dans lequel :

- r nombre entier peut prendre les valeurs 0, 1, 2,
- $R_3$ représente :
  - a) un atome d'hydrogène,
  - b) un alkyle linéaire ou ramifié de 1 à 6 atomes de carbone éventuellement substitué par
    * un ou deux groupements choisis parmi :
      - COOH, $-CO-R_6$, $-CO-O-R_6$ avec $R_6$ étant un groupement choisi parmi : alkyle linéaire ou ramifié saturé, insaturé, ou polyinsaturé de 1 à 12 atomes de carbone, et $-(CH_2)_m$-aryle éventuellement substitué où m, nombre entier, peut prendre les valeurs 0, 1, 2, ou 3,
    * ou par un groupement choisi parmi :
      i ) -OH, $-O-R_6$, $-O-CO-R_6$, ou $-O-CO-O-R_6$ avec $R_6$ tel que défini précédemment,
      ii) $-NR_7R_8$ avec $R_7$ et $R_8$, identiques ou différents, représentant chacun indépendamment l'un de l'autre un atome d'hydrogène, un alkyle linéaire ou ramifié de 1 à 6 atomes de carbone, $-CO-R_6$ ou $-CO-O-R_6$ avec $R_6$ ayant la même signification que précédemment, et $-(CH_2)_m$-aryle éventuellement substitué, où m nombre entier peut prendre les valeurs 0, 1, 2, ou 3,
      iii) $-CO-NR_9R_{10}$ avec $R_9$ et $R_{10}$, identiques ou différents, ayant la même définition que $R_6$ avec $R_6$ tel que défini précedemment ou pouvant également représenter un atome d'hydrogène, ou $R_9$ et $R_{10}$ forment ensemble, avec l'atome d'azote qui les porte, un système cyclique de 5 à 7 sommets pouvant comprendre éventuellement un second hétéroatome choisi parmi oxygène, azote, et soufre,
      iv) -SH, ou -SeH,
      v) un groupement $-S-R_6$, $-Se-R_6$, $-S-CO-O-R_6$,$-S-S-R_6$ ou $-Se-Se-R_6$ avec $R_6$ tel que défini précédemment,
      vi) un radical guanidino non substitué ou substitué par 1 à 2 groupements choisi parmi nitro et $-CO-O-R_6$ avec $R_6$ tel que défini précédemment,
      vii) un groupement indole ou imidazole éventuellement substitué,
  . c) un groupement $-(CH_2)_m$-phényle éventuellement substitué où m, nombre entier, peut prendre les valeurs 0, 1, 2, ou 3,
  étant entendu que $R_3$ ne peut pas représenter un groupement isobutyle,
- $R_4$ représente un atome d'hydrogène, un alkyle linéaire ou ramifié de 1 à 6 atomes de carbone, un groupement $-(CH_2)_p$-aryle éventuellement substitué où p, nombre entier peut prendre les valeurs 0, 1, 2, ou 3, ou un groupement amidino non substitué ou substitué par un

groupement nitro ou -CO-O-$R_6$ avec $R_6$ tel que défini précédemment, ou $R_4$ forme avec $R_3$ et les atomes auxquels ils sont liés un système mono ou bicyclique choisi parmi : pyrrolidine, pipéridine, perhydroindole, indoline, 2-aza bicyclo[2, 2, 2]octane, et 2-aza bicyclo[3, 3, 0]-octane, ces systèmes cycliques pouvant être éventuellement substitués par un ou plusieurs groupements choisis parmi :

hydroxy, oxo, alkyle linéaire ou ramifié de 1 à 6 atomes de carbone, alcoxy linéaire ou ramifié de 1 à 6 atomes de carbone,

avec la réserve que $R_3$ et $R_4$ ne peuvent pas former ensemble, avec les atomes auxquels ils sont liés, une pyrrolidine substituée par un groupement oxo en $\alpha$ de l'azote,

- $R_5$ représente :
  - un atome d'hydrogène,
  - un groupement $-(CH_2)_q$-aryle éventuellement substitué où q, nombre entier, peut prendre les valeurs 0, 1, 2, ou 3,
  - une chaine alkyle de 1 à 20 atomes de carbone, linéaire ou ramifiée, saturée, insaturée ou polyinsaturée, éventuellement interrompue par un ou plusieurs atomes d'oxygène, de soufre, ou d'azote, et éventuellement substituée par un ou plusieurs radicaux hydroxy, amino, alkylamino linéaire ou ramifié de 1 à 6 atomes de carbone, ou alcoxy linéaire ou ramifié de 1 à 6 atomes de carbone,
  - un groupement -CO-$R_{11}$ ou -CO-O-$R_{11}$ où $R_{11}$ représente :
    . une chaine alkyle de 1 à 20 atomes de carbone, linéaire ou ramifiée, saturée, insaturée ou polyinsaturée, éventuellement interrompue par un ou plusieurs atomes d'oxygène, de soufre, ou d'azote, et éventuellement substituée par un ou plusieurs radicaux hydroxy, amino, alkylamino linéaire ou ramifié de 1 à 6 atomes de carbone, alcoxy linéaire ou ramifié de 1 à 6 atomes de carbone, guanidino, ou guanidino substitué par 1 à 2 groupements choisis parmi nitro et -CO-O-$R_6$ avec $R_6$ tel que défini précédemment,
    . un groupement $-(CH_2)_q$-aryle éventuellement substitué où q, nombre entier, peut prendre les valeurs 0, 1, 2, ou 3,

étant entendu que, sauf précisions contraires, :

- le terme "aryle" désigne les radicaux phényle et naphtyle,
- le terme "substitué" associé aux expressions "$(CH_2)_m$-phényle", "$-(CH_2)_m$-aryle", "$-(CH_2)_p$-aryle", "$-(CH_2)_q$-aryle", "imidazole", et "indole" signifie que ces radicaux peuvent être substitués sur le cycle par un ou plusieurs groupements choisis parmi : hydroxy, halogène, nitro, trifluorométhyle, alkyle linéaire ou ramifié de 1 à 6 atomes de carbone, alcoxy linéaire ou ramifié de 1 à 6 atomes de carbone, $-(CH_2(_t$-phényle, -O-$(CH_2)_t$-phényle, et O-CO-O-$(CH_2)_t$-phényl, où t, nombre entier, peut prendre les valeurs 0, 1, 2, ou 3,
- et de leurs isomères, diastéréoisomères, épimères, isolés ou sous forme de mélange ainsi que, le cas échéant, de leurs sels d'addition à un acide ou à une base pharmaceutiquement acceptable,

caractérisé en ce que:

on condense, en utilisant les techniques de couplage passant par la formation d'un ester activé (hydroxy succinimide, hydroxybenzotriazole, en présence de dicyclohexyl carbodiimide), un composé de formule (II) :

HOOC - $R'_2$ (II)

dans laquelle $R'_2$ a la même signification que $R_2$ dans la formule (I) avec la réserve que lorsque $R'_2$ représente un groupement

$$-\underset{\underset{R_3}{|}}{CH} - (CH_2)r - NR_4R_5$$

tel que défini dans la formule (I) et que $R_4$ représente un atome d'hydrogène, alors $R_5$ représente un groupement -CO-$R_{11}$ ou -CO-O-$R_{11}$ avec $R_{11}$ ayant la même signification que dans la formule (I), avec une (alcoxybenzyl) pipérazine de formule (III) :

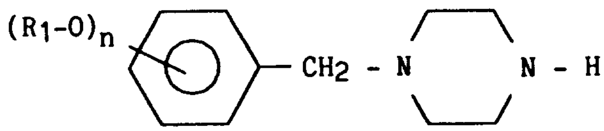

(III)

dans laquelle $R_1$ et n sont tels que définis dans la formule (I) afin d'obtenir un composé de formule (I/a) :

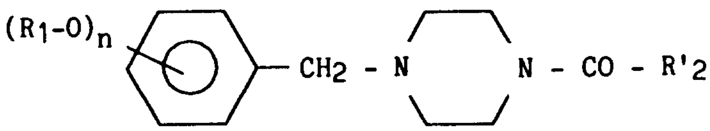

(I/a)

dans laquelle $R_1$, $R'_2$ et n sont tels que définis précédemment,
composés de formule (I/a) qui sont, dans le cas où $R_5$ représente un groupement $-CO-O-R_{11}$, éventuellement soumis si on le désire, aux réactions de déprotection des fonctions aminées en synthèse peptidique (traitement acide ou hydrogénation catalytique suivant la nature de $R_{11}$) afin d'accéder aux composés de formule (I) pour lesquels $R_5$ représente un hydrogène,
les composés de formule (I) pour lesquels $R_5$ représente un atome d'hydrogène pouvant ensuite, le cas échéant, être mis en réaction avec un composé de formule (IV) :

$HOOC-R_{11}$ (IV)

dans laquelle $R_{11}$ est tel que défini précédemment, afin d'obtenir un composé de formule (I/b) :

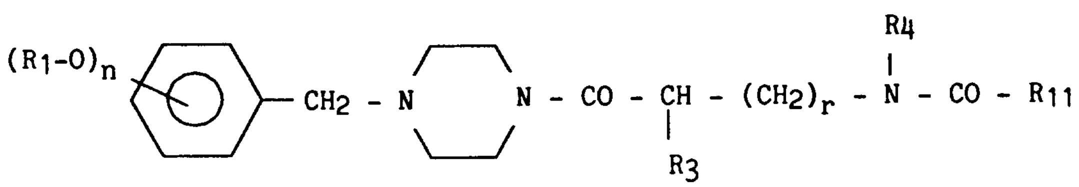

(I/b)

dans laquelle $R_1$, $R_3$, $R_4$, $R_{11}$ et n sont tels que définis précédemment,
cas particulier des composés de formule (I) dans lesquels $R_2$ représente un groupement de formule :

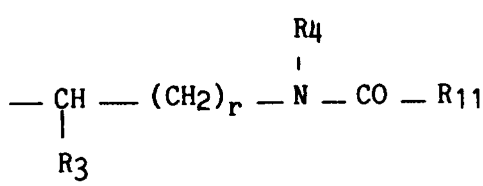

les composés de formule (I) pouvant, si nécessaire, être purifiés, séparés, le cas échéant, en leurs différents isomères optiques, et pouvant également être salifiés, soit avec un acide pharmaceutiquement acceptable, soit, si cela est possible et si on le désire avec une base pharmaceutiquement acceptable.

**2.** Procédé de préparation selon la revendication 1 des composés de formule (I) pour lesquels n est égal à 3 et $R_1$ représente un méthyle, les trois groupements méthoxy ainsi définis étant portés en positions 2, 3, et 4 par le noyau aromatique de la benzylpipérazine, ce qui correspond aux composés de formule générale (I $\alpha$)

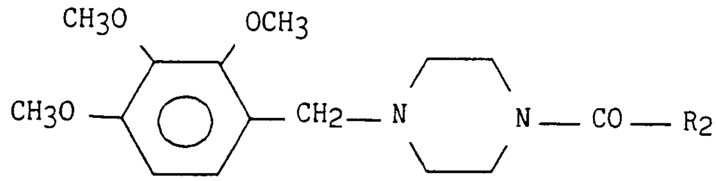 (I a)

dans laquelle $R_2$ est tel que défini dans la revendication 1 :

- de eurs isomères, diastéréoisomères, épimères isolés ou sous forme de mélange,
- et de leurs sels d'addition à un acide ou à une base pharmaceutiquement acceptable.

**3.** Procédé de préparation selon la revendication 1 des composés de formule (I) pour lesquels $R_2$ représente un groupement

$$-\underset{\displaystyle R_3}{\underset{|}{CH}}-(CH_2)_r-NR_4R_5$$

tel que défini dans la revendication 1, dans lequel $R_3$ représente spécifiquement un alkyle linéaire ou ramifié de 1 à 6 atomes de carbone substitué par un groupement -SH ou -S-$R_6$ avec $R_6$ tel que défini dans la revendication 1,

- de leurs isomères, diastéréoisomères, épimères, isolés ou sous forme de mélange
- et de leurs sels d'addition à un acide ou à une base pharmaceutiquement acceptable.

**4.** Procédé de préparation selon la revendication 1 des composés de formule (I) pour lesquels

- n est égal à 3,
- $R_1$ est un groupement méthyl, les trois groupements méthoxy ainsi définis étant portés en position 2, 3 et 4 par le noyau aromatique de la benzylpipérazine,
- $R_2$ est un groupement

$$-\underset{\displaystyle R_3}{\underset{|}{CH}}-(CH_2)_r-NR_4R_5$$

  tel que défini dans la revendication 1 pour lequel $R_3$ représente un alkyl linéaire ou ramifié de 1 à 6 atomes de carbone substitué par un groupement -SH ou -S-$R_6$ avec $R_6$ tel que défini dans la revendication 1,
- de leurs isomères, diastéréoisomères, épimères isolés ou sous forme de mélange,
- et de leurs sels d'addition à un acide ou à une base pharmaceutiquement acceptable.

**5.** Procédé de préparation selon la revendication 1 des composés de formule (I) pour lesquels $R_2$ représente un groupement

$$-\underset{\displaystyle R_3}{\underset{|}{CH}}-(CH_2)_r-NR_4R_5$$

tel que défini dans la revendication 1, dans lequel $R_3$ et $R_4$ forment ensemble, avec les atomes auxquels ils sont liés un système mono ou bicyclique choisi parmi : pyrrolidine, pipéridine, perhydroindole, indoline, 2-aza bicyclo[2, 2, 2] octane, et 2-aza bicyclo[3, 3, 0] octane, ces systèmes cycliques pouvant être éventuellement substitués par un ou plusieurs groupements choisis parmi :

hydroxy, oxo, alkyle linéaire ou ramifié de 1 à 6 atomes de carbone, alcoxy linéaire ou ramifié de 1 à 6 atomes de carbone,

avec la réserve que $R_3$ et $R_4$ ne peuvent pas former ensemble, avec les atomes auxquels ils sont liés, une pyrrolidine substituée par un groupement oxo en $\alpha$ de l'azote,

- de leurs isomères, diastéréoisomères, épimères, isolés ou sous forme de mélange,
- et de leurs sels d'addition à un acide ou à une base pharmaceutiquement acceptable.

**6.** Procédé de préparation selon la revendication 1 des composés de formule (I) pour lesquels $R_3$ représente un alkyle linéaire ou ramifié de 1 à 6 atomes de carbone éventuellement substitué par un radical guanidino non substitué ou substitué par 1 à 2 groupements choisis parmi nitro et -CO-O-$R_6$ avec $R_6$ tel que défini dans la revendication 1,

- de leurs isomères, diastéréoisomères, épimères, isolés ou sous forme de mélange,
- et de leurs sels d'addition à un acide ou à une base pharmaceutiquement acceptable.

**7.** Procédé de préparation selon la revendication 1 du composé qui est la 1-(2,3,4-triméthoxybenzyl) 4-[S-benzyl N-benzyloxycarbonyl L-cystéinyl] pipérazine, de ses isomères optiques, isolés ou sous forme de mélange, et de ses sels d'addition à un acide pharmaceutiquement acceptable.

**8.** Procédé de préparation selon la revendication 1 du composé qui est la 1-(2,3,4-triméthoxybenzyl) 4-[N-benzyloxycarbonyl L-alanyl] pipérazine, de ses isomères optiques, isolés ou sous forme de mélange, et de ses sels d'addition à un acide pharmaceutiquement acceptable.

**9.** Procédé de préparation selon la revendication 1 du composé qui est la 1-(2,3,4-triméthoxybenzyl) 4-glycyl pipérazine et de ses sels d'addition à un acide pharmaceutiquement acceptable.

**10.** Procédé de préparation selon la revendication 1 du composé qui est la 1-(2,3,4-triméthoxybenzyl) 4-[N-benzyloxycarbonyl L-prolyl] pipérazine, de ses isomères optiques, isolés ou sous forme de mélange, et de ses sels d'addition à un acide pharmaceutiquement acceptable.

**11.** Procédé de préparation selon la revendication 1 du composé qui est la 1-(2,3,4-triméthoxybenzyl) 4-[S-benzyl L-cystéinyl] pipérazine, de ses isomères optiques, isolés ou sous forme de mélange, et de ses sels d'addition à un acide pharmaceutiquement acceptable.

**12.** Procédé de préparation selon la revendication 1 du composé qui est la 1-(2,3,4-triméthoxybenzyl) 4-(L-arginyl) pipérazine, de ses isomères optiques, isolés ou sous forme de mélange, et de ses sels d'addition à un acide pharmaceutiquement acceptable.

**13.** Procédé de préparation selon la revendication 1 du composé qui est la 1-(2,3,4-triméthoxybenzyl) 4-[$N_\alpha$, $N_\delta$, $N_\omega$-tribenzoloxycarbonyl L-arginyl) pipérazine, de ses isomères optiques, isolés ou sous forme de mélange, et de ses sels d'addition à un acide pharmaceutiquement acceptable.

**14.** Procédé de préparation selon la reven du composé qui est la 1-(2,3,4-triméthoxybenzyl) 4-[2-amino 4,4-dicarboxybutyryl] pipérazine, de ses isomères optiques, isolés ou sous forme de mélange, et de ses sels d'addition à un acide pharmaceutiquement acceptable.

**15.** Procédé de préparation d'une composition pharmaceutique contenant comme principe actif au moins un composé préparé selon l'une des revendications 1 à 14 en combinaison avec un ou plusieurs excipients ou véhicules pharmaceutiquement acceptables.

**16.** Procédé de préparation selon la revendication 15 d'une composition pharmaceutique, utilisable pour le traitement et la prévention de l'ischémie cérébrale, de l'hypoxie vasculaire cérébrale, de l'anoxie vasculaire cérébrale et des oedèmes cérébraux, pour le traitement des accidents vasculaires cérébraux, des traumatismes crâniens, des encéphalopathies, des maladie neuro-dégénératives et des troubles de la sénescence, ainsi que pour le traitement et la prévention de l'ischémie de type périphérique, en cardiologie : l'ischémie myocardique et l'ischémie coronarienne et leurs différentes expressions cliniques : angine de poitrine, infarctus du myocarde, troubles du rythme, spasme vasculaire, insuffisance cardiaque, ainsi qu'en ophtamologie et en oto-rhino-laryngologie lors d'atteintes vasculaires chorio-rétiniennes, de vertiges d'origine vasculaire, de vertiges de Menière ou d'acouphènes.

## Claims

**Claims for the following Contracting States : AT, BE, CH, LI, DE, DK, FR, GB, IT, LU, NL, PT, SE, IE**

**1.** Compounds of the general formula (I):

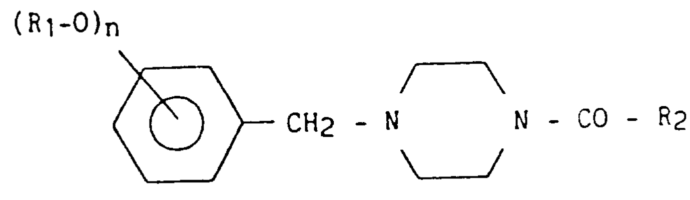

(I)

wherein:

- n, which is an integer, may have the value 1, 2 or 3,
- $R_1$ is a linear or branched alkyl containing from 1 to 4 carbon atoms,
- $R_2$ represents:
  a group

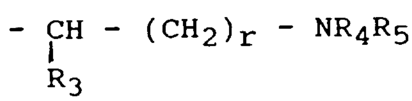

  wherein:
  - r, which is an integer, may have the value 0, 1 or 2,
  - $R_3$ represents:
    - a) a hydrogen atom,
    - b) a linear or branched alkyl having from 1 to 6 carbon atoms that is optionally substituted by
      * one or two groups selected from:
        - COOH, $-CO-R_6$, $-CO-O-R_6$, with $R_6$ being a group selected from: linear or branched, saturated, unsaturated or polyunsaturated alkyl having from 1 to 12 carbon atoms, and optionally substituted $-(CH_2)_m$-aryl wherein m, which is an integer, may have the value 0, 1, 2 or 3,
      * or by a group selected from:
        i) -OH, $-O-R_6$, $-O-CO-R_6$ or $-O-CO-O-R_6$ wherein $R_6$ is as defined above,
        ii) $-NR_7R_8$ wherein each of $R_7$ and $R_8$, which may be identical or different, represents independently of the other a hydrogen atom, a linear or branched alkyl having from 1 to 6 carbon atoms, $-CO-R_6$ or $-CO-O-R_6$ with $R_6$ being as defined above, and optionally substituted $-(CH_2)_m$-aryl wherein m, which is an integer, may have the value 0, 1, 2 or 3,
        iii) $-CO-NR_9R_{10}$ wherein $R_9$ and $R_{10}$, which may be identical or different, are as defined for $R_6$, with $R_6$ being as defined above, or may equally represent a hydrogen atom, or $R_9$ and $R_{10}$ together form, with the nitrogen atom carrying them, a cyclic system having from 5 to 7 ring members which may optionally contain a second hetero atom selected from oxygen, nitrogen and sulphur,
        iv) -SH or -SeH,
        v) a $-S-R_6$, $-Se-R_6$, $-S-CO-O-R_6$, $-S-S-R_6$ or $-Se-Se-R_6$ group wherein $R_6$ is as defined above,
        vi) a guanidino radical that is unsubstituted or substituted by 1 or 2 groups selected from nitro and $-CO-O-R_6$ wherein $R_6$ is as defined above,
        vii) an optionally substituted indole or imidazole group,
    - c) an optionally substituted $-(CH_2)_m$-phenyl group wherein m, which is an integer, may have the value 0, 1, 2 or 3,

    it being understood that $R_3$ cannot represent an isobutyl group,

- $R_4$ represents a hydrogen atom, a linear or branched alkyl having from 1 to 6 carbon atoms, an optionally substituted $-(CH_2)_p$-aryl group wherein p, which is an integer, may have the value 0, 1, 2 or 3, or an amidino group that is unsubstituted or substituted by a nitro or -CO-O-$R_6$ group wherein $R_6$ is as defined above,

  or $R_4$ forms with $R_3$ and the atoms to which they are bonded a mono- or bi-cyclic system selected from: pyrrolidine, piperidine, perhydroindole, indoline, 2-azabicyclo[2.2.2]octane and 2-azabicyclo[3.3.0]octane, which cyclic systems may optionally be substituted by one or more groups selected from:

  hydroxy, oxo, linear or branched alkyl having from 1 to 6 carbon atoms, linear or branched alkoxy having from 1 to 6 carbon atoms,

with the proviso that $R_3$ and $R_4$ cannot together form, with the atoms to which they are bonded, a pyrrolidine substituted by an oxo group $\alpha$ to the nitrogen,

- $R_5$ represents:
  - a hydrogen atom,
  - an optionally substituted $-(CH_2)_q$-aryl group wherein q, which is an integer, may have the value 0, 1, 2 or 3,
  - a linear or branched, saturated, unsaturated or polyunsaturated alkyl chain having from 1 to 20 carbon atoms that is optionally interrupted by one or more oxygen, sulphur or nitrogen atoms and is optionally substituted by one or more radicals hydroxy, amino, linear or branched alkylamino having from 1 to 6 carbon atoms, or linear or branched alkoxy having from 1 to 6 carbon atoms,
  - a -CO-$R_{11}$ or -CO-O-$R_{11}$ group wherein $R_{11}$ represents:
    . a linear or branched, saturated, unsaturated or polyunsaturated alkyl chain having from 1 to 20 carbon atoms that is optionally interrupted by one or more oxygen, sulphur or nitrogen atoms and is optionally substituted by one or more radicals hydroxy, amino, linear or branched alkylamino having from 1 to 6 carbon atoms, linear or branched alkoxy having from 1 to 6 carbon atoms, guanidino, or guanidino substituted by 1 or 2 groups selected from nitro and -CO-O-$R_6$ wherein $R_6$ is as defined above,
    . an optionally substituted $-(CH_2)_q$-aryl group wherein q, which is an integer, may have the value 0, 1, 2 or 3,

it being understood that, unless specified to the contrary:

- the term "aryl" denotes phenyl and naphthyl radicals,
- the term "substituted" associated with the terms "$(CH_2)_m$-phenyl", "$-(CH_2)_m$-aryl", "$-(CH_2)_p$-aryl", "$-(CH_2)_q$-aryl", "imidazole" and "indole" denotes that those radicals may be substituted on the ring by one or more groups selected from: hydroxy, halogen, nitro, trifluoromethyl, linear or branched alkyl having from 1 to 6 carbon atoms, linear or branched alkoxy having from 1 to 6 carbon atoms, $-(CH_2)_t$-phenyl, $-O-(CH_2)_t$-phenyl and $O-CO-O-(CH_2)_t$-phenyl, wherein t, which is an integer, may have the value 0, 1, 2 or 3,
- and their isomers, diastereoisomers, epimers, isolated or in the form of a mixture, and also, where appropriate, their addition salts with a pharmaceutically acceptable acid or base.

**2.** Compounds according to claim 1 wherein n is 3 and $R_1$ represents methyl, the three methoxy groups so defined being carried in positions 2, 3 and 4 on the aromatic ring of the benzylpiperazine, which corresponds to the compounds of the general formula (Iα)

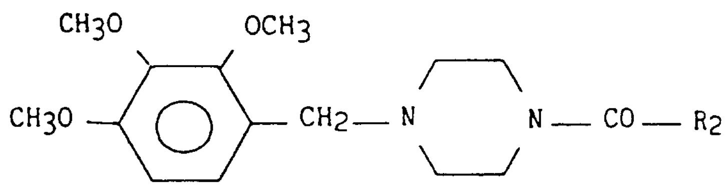

(Iα)

wherein $R_2$ is as defined in claim 1:

- their isomers, diastereoisomers, epimers, isolated or in the form of a mixture,
- and their addition salts with a pharmaceutically acceptable acid or base.

**3.** Compounds according to claim 1 wherein $R_2$ represents a group

$$-\underset{\displaystyle R_3}{\underset{|}{CH}} - (CH_2)_r - NR_4R_5$$

as defined in claim 1, wherein $R_3$ represents specifically a linear or branched alkyl having from 1 to 6 carbon atoms that is substituted by a -SH or -S-$R_6$ group wherein $R_6$ is as defined in claim 1,
- their isomers, diastereoisomers, epimers, isolated or in the form of a mixture,
- and their addition salts with a pharmaceutically acceptable acid or base.

**4.** Compounds according to claim 1 wherein
- n is 3,
- $R_1$ is a methyl group, the three methoxy groups so defined being carried in positions 2, 3 and 4 on the aromatic ring of the benzylpiperazine,
- $R_2$ is a group

$$-\underset{\displaystyle R_3}{\underset{|}{CH}} - (CH_2)_r - NR_4R_5$$

  as defined in claim 1, wherein $R_3$ represents a linear or branched alkyl having from 1 to 6 carbon atoms that is substituted by a -SH or -S-$R_6$ group wherein $R_6$ is as defined in claim 1,
- their isomers, diastereoisomers, epimers, isolated or in the form of a mixture,
- and their addition salts with a pharmaceutically acceptable acid or base.

**5.** Compounds according to claim 1 wherein $R_2$ represents a group

$$-\underset{\displaystyle R_3}{\underset{|}{CH}} - (CH_2)_r - NR_4R_5$$

as defined in claim 1, wherein $R_3$ and $R_4$ together form, with the atoms to which they are bonded, a mono- or bi-cyclic system selected from: pyrrolidine, piperidine, perhydroindole, indoline, 2-azabicyclo-[2.2.2]octane and 2-azabicyclo[3.3.0]octane, which cyclic systems may optionally be substituted by one or more groups selected from:

hydroxy, oxo, linear or branched alkyl having from 1 to 6 carbon atoms, linear or branched alkoxy having from 1 to 6 carbon atoms,

with the proviso that $R_3$ and $R_4$ cannot together form, with the atoms to which they are bonded, a pyrrolidine substituted by an oxo group $\alpha$ to the nitrogen,
- their isomers, diastereoisomers, epimers, isolated or in the form of a mixture,
- and their addition salts with a pharmaceutically acceptable acid or base.

**6.** Compounds according to claim 1 wherein $R_3$ represents a linear or branched alkyl having from 1 to 6 carbon atoms that is optionally substituted by a guanidino radical that is unsubstituted or substituted by 1 or 2 groups selected from nitro and -CO-O-$R_6$ wherein $R_6$ is as defined in claim 1,
- their isomers, diastereoisomers, epimers, isolated or in the form of a mixture,
- and their addition salts with a pharmaceutically acceptable acid or base.

**7.** The compound according to claim 1 that is 1-(2,3,4-trimethoxybenzyl)-4-[S-benzyl-N-benzyloxycarbonyl-L-cysteinyl]piperazine, its optical isomers, isolated or in the form of a mixture, and its addition salts with a pharmaceutically acceptable acid.

**8.** The compound according to claim 1 that is 1-(2,3,4-trimethoxybenzyl)-4-[N-benzyloxycarbonyl-L-alanyl]piperazine, its optical isomers, isolated or in the form of a mixture, and its addition salts with a

pharmaceutically acceptable acid.

**9.** The compound according to claim 1 that is 1-(2,3,4-trimethoxybenzyl)-4-glycylpiperazine and its addition salts with a pharmaceutically acceptable acid.

**10.** The compound according to claim 1 that is 1-(2,3,4-trimethoxybenzyl)-4-[N-benzyloxycarbonyl-L-prolyl]-piperazine, its optical isomers, isolated or in the form of a mixture, and its addition salts with a pharmaceutically acceptable acid.

**11.** The compound according to claim 1 that is 1-(2,3,4-trimethoxybenzyl)-4-[S-benzyl-L-cysteinyl]-piperazine, its optical isomers, isolated or in the form of a mixture, and its addition salts with a pharmaceutically acceptable acid.

**12.** The compound according to claim 1 that is 1-(2,3,4-trimethoxybenzyl)-4-(L-arginyl)piperazine, its optical isomers, isolated or in the form of a mixture, and its addition salts with a pharmaceutically acceptable acid.

**13.** The compound according to claim 1 that is 1-(2,3,4-trimethoxybenzyl)-4-[$N_\alpha$,$N_\delta$,$N_\omega$-tribenzyloxycarbonyl-L-arginyl]piperazine, its optical isomers, isolated or in the form of a mixture, and its addition salts with a pharmaceutically acceptable acid.

**14.** The compound according to claim 1 that is 1-(2,3,4-trimethoxybenzyl)-4-[2-amino-4,4-dicarboxybutyryl]-piperazine, its optical isomers, isolated or in the form of a mixture, and its addition salts with a pharmaceutically acceptable acid.

**15.** Process for the preparation of the compounds of formula (I) according to claim 1, characterised in that, using coupling techniques proceeding <u>via</u> the formation of an activated ester (hydroxysuccinimide, hydroxybenzotriazole, in the presence of dicyclohexylcarbodiimide) a compound of formula (II):

$HOOC - R'_2$ (II)

wherein $R'_2$ is as defined for $R_2$ in claim 1, with the proviso that when $R'_2$ represents a group

$$-\underset{\displaystyle R_3}{\underset{|}{CH}} - (CH_2)_r - NR_4R_5$$

as defined in claim 1 and $R_4$ represents a hydrogen atom $R_5$ represents a group $-CO-R_{11}$ or $-CO-O-R_{11}$ wherein $R_{11}$ is as defined in claim 1, is condensed
with an (alkoxybenzyl)piperazine of formula (III):

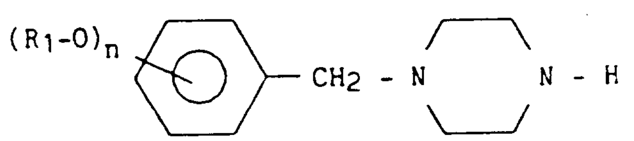
(III),

wherein $R_1$ and <u>n</u> are as defined in formula (I), in order to obtain a compound of formula (I/a):

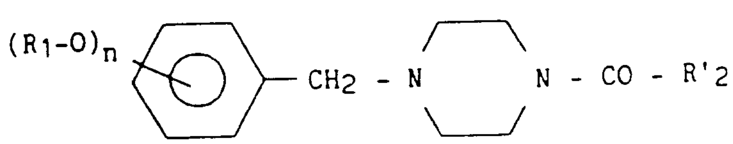
(I/a)

wherein $R_1$, $R'_2$ and n are as defined above,
which compounds of formula (I/a), when $R_5$ represents a group -CO-O-$R_{11}$, are optionally subjected, if desired, to reactions of peptide synthesis deprotecting the amine functions (acid treatment or catalytic hydrogenation depending on the nature of $R_{11}$) in order to obtain the compounds of formula (I) according to claim 1 wherein $R_5$ represents a hydrogen atom,
which compounds of formula (I) according to claim 1 wherein $R_5$ represents a hydrogen atom may then, if desired, be reacted with a compound of formula (IV):

HOOC-$R_{11}$ (IV),

wherein $R_{11}$ is as defined above, in order to obtain a compound of formula (I/b):

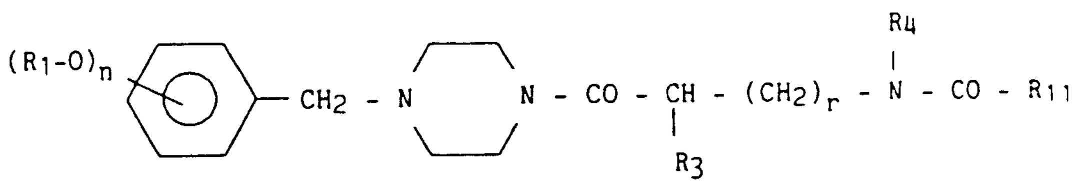 (I/b)

wherein $R_1$, $R_3$, $R_4$, $R_{11}$ and n are as defined above,
a particular case of the compounds of formula (I) according to claim 1 wherein $R_2$ represents a group of the formula:

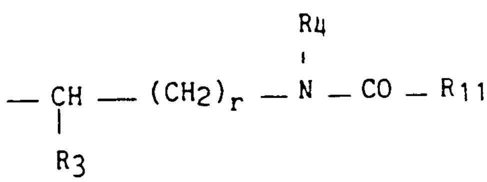

which compounds of formula (I) according to claim 1 may, if necessary, be purified, separated, where appropriate, into their different optical isomers and may also be converted into salts, either with a pharmaceutically acceptable acid or, if possible and if desired, with a pharmaceutically acceptable base.

**16.** Pharmaceutical composition containing as active ingredient at least one compound according to one of claims 1 to 14 in combination with one or more pharmaceutically acceptable excipients or carriers.

**17.** Pharmaceutical composition according to claim 16, for use in the treatment and prevention of cerebral ischaemia, cerebral vascular hypoxia, cerebral vascular anoxia and cerebral oedema, in the treatment of cerebral vascular accidents, cerebral trauma, encephalopathies, neurodegenerative diseases and disorders associated with ageing, and also in the treatment and prevention of ischaemia of the peripheral type, in cardiology: myocardial ischaemia and coronary ischaemia and their various clinical manifestations: angina pectoris, myocardial infarct, rhythm disorders, vascular spasm, cardiac insufficiency, and also in ophthalmology and in otorhinolaryngology in the case of vascular chorio-retinitis episodes, vertigo of vascular origin, Meniere's vertigo or other vertigos of inner ear origin.

**Claims for the following Contracting States : ES, GR**

**1.** Process for the preparation of compounds of the general formula (I):

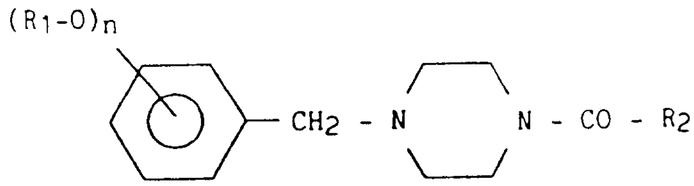

(I)

wherein:

- n, which is an integer, may have the value 1, 2 or 3,
- $R_1$ is a linear or branched alkyl containing from 1 to 4 carbon atoms,
- $R_2$ represents:
  a group

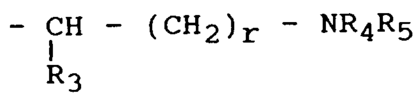

  wherein:
  - r, which is an integer, may have the value 0, 1 or 2,
  - $R_3$ represents:
    - a) a hydrogen atom,
    - b) a linear or branched alkyl having from 1 to 6 carbon atoms that is optionally substituted by
      * one or two groups selected from:
        - COOH, $-CO-R_6$, $-CO-O-R_6$, with $R_6$ being a group selected from: linear or branched, saturated, unsaturated or polyunsaturated alkyl having from 1 to 12 carbon atoms, and optionally substituted $-(CH_2)_m$-aryl wherein m, which is an integer, may have the value 0, 1, 2 or 3,
      * or by a group selected from:
        i) -OH, $-O-R_6$, $-O-CO-R_6$ or $-O-CO-O-R_6$ wherein $R_6$ is as defined above,
        ii) $-NR_7R_8$ wherein each of $R_7$ and $R_8$, which may be identical or different, represents independently of the other a hydrogen atom, a linear or branched alkyl having from 1 to 6 carbon atoms, $-CO-R_6$ or $-CO-O-R_6$ with $R_6$ being as defined above, and optionally substituted $-(CH_2)_m$-aryl wherein m, which is an integer, may have the value 0, 1, 2 or 3,
        iii) $-CO-NR_9R_{10}$ wherein $R_9$ and $R_{10}$, which may be identical or different, are as defined for $R_6$, with $R_6$ being as defined above, or may equally represent a hydrogen atom, or $R_9$ and $R_{10}$ together form, with the nitrogen atom carrying them, a cyclic system having from 5 to 7 ring members which may optionally contain a second hetero atom selected from oxygen, nitrogen and sulphur,
        iv) -SH or -SeH,
        v) a $-S-R_6$, $-Se-R_6$, $-S-CO-O-R_6$, $-S-S-R_6$ or $-Se-Se-R_6$ group wherein $R_6$ is as defined above,
        vi) a guanidino radical that is unsubstituted or substituted by 1 or 2 groups selected from nitro and $-CO-O-R_6$ wherein $R_6$ is as defined above,
        vii) an optionally substituted indole or imidazole group,
    - c) an optionally substituted $-(CH_2)_m$-phenyl group wherein m, which is an integer, may have the value 0, 1, 2 or 3,

    it being understood that $R_3$ cannot represent an isobutyl group,
  - $R_4$ represents a hydrogen atom, a linear or branched alkyl having from 1 to 6 carbon atoms, an optionally substituted $-(CH_2)_p$-aryl group wherein p, which is an integer, may have the value 0, 1, 2 or 3, or an amidino group that is unsubstituted or substituted by a nitro or $-CO-O-R_6$ group wherein $R_6$ is as defined above,

    or $R_4$ forms with $R_3$ and the atoms to which they are bonded a mono- or bi-cyclic system

selected from: pyrrolidine, piperidine, perhydroindole, indoline, 2-azabicyclo[2.2.2]octane and 2-azabicyclo[3.3.0]octane, which cyclic systems may optionally be substituted by one or more groups selected from:

hydroxy, oxo, linear or branched alkyl having from 1 to 6 carbon atoms, linear or branched alkoxy having from 1 to 6 carbon atoms,

with the proviso that $R_3$ and $R_4$ cannot together form, with the atoms to which they are bonded, a pyrrolidine substituted by an oxo group $\alpha$ to the nitrogen,

- $R_5$ represents:
  - a hydrogen atom,
  - an optionally substituted $-(CH_2)_q$-aryl group wherein q, which is an integer, may have the value 0, 1, 2 or 3,
  - a linear or branched, saturated, unsaturated or polyunsaturated alkyl chain having from 1 to 20 carbon atoms that is optionally interrupted by one or more oxygen, sulphur or nitrogen atoms and is optionally substituted by one or more radicals hydroxy, amino, linear or branched alkylamino having from 1 to 6 carbon atoms, or linear or branched alkoxy having from 1 to 6 carbon atoms,
  - a $-CO-R_{11}$ or $-CO-O-R_{11}$ group wherein $R_{11}$ represents:
    . a linear or branched, saturated, unsaturated or polyunsaturated alkyl chain having from 1 to 20 carbon atoms that is optionally interrupted by one or more oxygen, sulphur or nitrogen atoms and is optionally substituted by one or more radicals hydroxy, amino, linear or branched alkylamino having from 1 to 6 carbon atoms, linear or branched alkoxy having from 1 to 6 carbon atoms, guanidino, or guanidino substituted by 1 or 2 groups selected from nitro and $-CO-O-R_6$ wherein $R_6$ is as defined above,
    . an optionally substituted $-(CH_2)_q$-aryl group wherein q, which is an integer, may have the value 0, 1, 2 or 3,

it being understood that, unless specified to the contrary:

- the term "aryl" denotes phenyl and naphthyl radicals,
- the term "substituted" associated with the terms "$(CH_2)_m$-phenyl", "$-(CH_2)_m$-aryl", "$-(CH_2)_p$-aryl", "$-(CH_2)_q$-aryl", "imidazole" and "indole" denotes that those radicals may be substituted on the ring by one or more groups selected from: hydroxy, halogen, nitro, trifluoromethyl, linear or branched alkyl having from 1 to 6 carbon atoms, linear or branched alkoxy having from 1 to 6 carbon atoms, $-(CH_2)_t$-phenyl, $-O-(CH_2)_t$-phenyl and $O-CO-O-(CH_2)_t$-phenyl, wherein t, which is an integer, may have the value 0, 1, 2 or 3,
- and their isomers, diastereoisomers, epimers, isolated or in the form of a mixture, and also, where appropriate, their addition salts with a pharmaceutically acceptable acid or base,

characterised in that:

using coupling techniques proceeding via the formation of an activated ester (hydroxysuccinimide, hydroxybenzotriazole, in the presence of dicyclohexylcarbodiimide) a compound of formula (II):

$HOOC - R'_2$ (II)

wherein $R'_2$ is as defined for $R_2$ in formula (I), with the proviso that when $R'_2$ represents a group

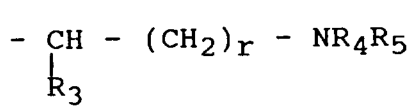

as defined in formula (I) and $R_4$ represents a hydrogen atom $R_5$ represents a group $-CO-R_{11}$ or $-CO-O-R_{11}$ wherein $R_{11}$ is as defined in formula (I), is condensed with an (alkoxybenzyl)piperazine of formula (III):

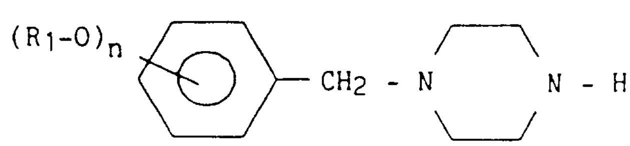 (III),

wherein $R_1$ and n are as defined in formula (I), in order to obtain a compound of formula (I/a):

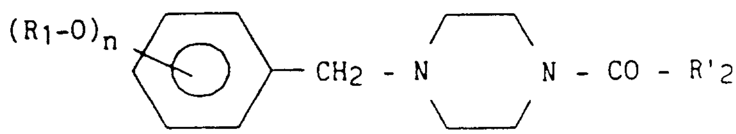 (I/a)

wherein $R_1$, $R'_2$ and n are as defined above,
which compounds of formula (I/a), when $R_5$ represents a group -CO-O-$R_{11}$, are optionally subjected, if desired, to reactions of peptide synthesis deprotecting the amine functions (acid treatment or catalytic hydrogenation depending on the nature of $R_{11}$) in order to obtain the compounds of formula (I) wherein $R_5$ represents a hydrogen atom,
which compounds of formula (I) wherein $R_5$ represents a hydrogen atom may then, if desired, be reacted with a compound of formula (IV):

HOOC-$R_{11}$ (IV),

wherein $R_{11}$ is as defined above, in order to obtain a compound of formula (I/b):

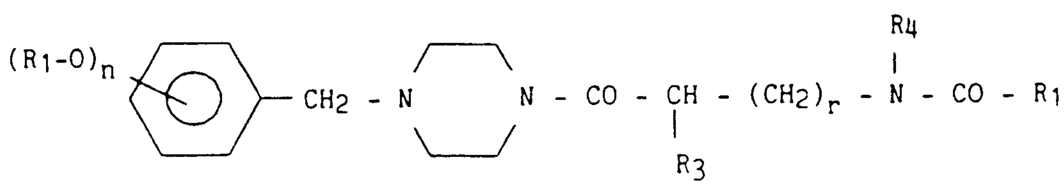 (I/b)

wherein $R_1$, $R_3$, $R_4$, $R_{11}$ and n are as defined above,
a particular case of the compounds of formula (I) wherein $R_2$ represents a group of the formula:

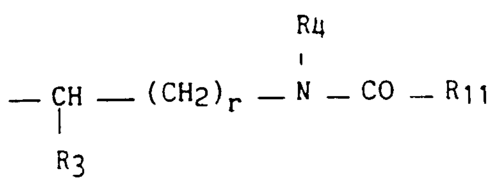

which compounds of formula (I) may, if necessary, be purified, separated, where appropriate, into their different optical isomers and may also be converted into salts, either with a pharmaceutically acceptable acid or, if possible and if desired, with a pharmaceutically acceptable base.

**2.** Process for the preparation according to claim 1 of compounds of formula (I) wherein n is 3 and $R_1$ represents methyl, the three methoxy groups so defined being carried in positions 2, 3 and 4 on the aromatic ring of the benzylpiperazine, which corresponds to the compounds of the general formula (Iα)

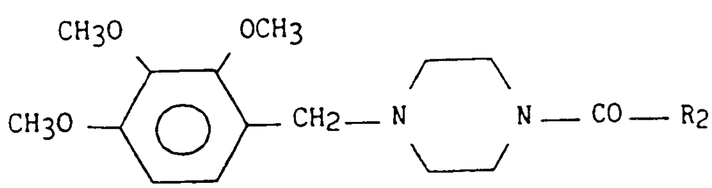 (Iα)

wherein $R_2$ is as defined in claim 1:
- their isomers, diastereoisomers, epimers, isolated or in the form of a mixture,
- and their addition salts with a pharmaceutically acceptable acid or base.

**3.** Process for the preparation according to claim 1 of compounds of formula (I) wherein $R_2$ represents a group

$$-\underset{\displaystyle R_3}{\underset{|}{CH}}-(CH_2)_r-NR_4R_5$$

as defined in claim 1,
wherein $R_3$ represents specifically a linear or branched alkyl having from 1 to 6 carbon atoms that is substituted by a -SH or -S-$R_6$ group wherein $R_6$ is as defined in claim 1,

- their isomers, diastereoisomers, epimers, isolated or in the form of a mixture,
- and their addition salts with a pharmaceutically acceptable acid or base.

**4.** Process for the preparation according to claim 1 of compounds of formula (I) wherein

- $\underline{n}$ is 3,
- $R_1$ is a methyl group, the three methoxy groups so defined being carried in positions 2, 3 and 4 on the aromatic ring of the benzylpiperazine,
- $R_2$ is a group

$$-\underset{\displaystyle R_3}{\underset{|}{CH}}-(CH_2)_r-NR_4R_5$$

  as defined in claim 1, wherein $R_3$ represents a linear or branched alkyl having from 1 to 6 carbon atoms that is substituted by a -SH or -S-$R_6$ group wherein $R_6$ is as defined in claim 1,
- their isomers, diastereoisomers, epimers, isolated or in the form of a mixture,
- and their addition salts with a pharmaceutically acceptable acid or base.

**5.** Process for the preparation according to claim 1 of compounds of formula (I) wherein $R_2$ represents a group

$$-\underset{\displaystyle R_3}{\underset{|}{CH}}-(CH_2)_r-NR_4R_5$$

as defined in claim 1,
wherein $R_3$ and $R_4$ together form, with the atoms to which they are bonded, a mono- or bi-cyclic system selected from: pyrrolidine, piperidine, perhydroindole, indoline, 2-azabicyclo[2.2.2]octane and 2-azabicyclo[3.3.0]octane, which cyclic systems may optionally be substituted by one or more groups selected from:
hydroxy, oxo, linear or branched alkyl having from 1 to 6 carbon atoms, linear or branched alkoxy having from 1 to 6 carbon atoms,
with the proviso that $R_3$ and $R_4$ cannot together form, with the atoms to which they are bonded, a pyrrolidine substituted by an oxo group $\alpha$ to the nitrogen,

- their isomers, diastereoisomers, epimers, isolated or in the form of a mixture,
- and their addition salts with a pharmaceutically acceptable acid or base.

**6.** Process for the preparation according to claim 1 of compounds of formula (I) wherein $R_3$ represents a linear or branched alkyl having from 1 to 6 carbon atoms that is optionally substituted by a guanidino radical that is unsubstituted or substituted by 1 or 2 groups selected from nitro and -CO-O-$R_6$ wherein $R_6$ is as defined in claim 1,

- their isomers, diastereoisomers, epimers, isolated or in the form of a mixture,
- and their addition salts with a pharmaceutically acceptable acid or base.

7. Process for the preparation according to claim 1 of the compound that is 1-(2,3,4-trimethoxybenzyl)-4-[S-benzyl-N-benzyloxycarbonyl-L-cysteinyl]piperazine, its optical isomers, isolated or in the form of a mixture, and its addition salts with a pharmaceutically acceptable acid.

8. Process for the preparation according to claim 1 of the compound that is 1-(2,3,4-trimethoxybenzyl)-4-[N-benzyloxycarbonyl-L-alanyl]piperazine, its optical isomers, isolated or in the form of a mixture, and its addition salts with a pharmaceutically acceptable acid.

9. Process for the preparation according to claim 1 of the compound that is 1-(2,3,4-trimethoxybenzyl)-4-glycylpiperazine and its addition salts with a pharmaceutically acceptable acid.

10. Process for the preparation according to claim 1 of the compound that is 1-(2,3,4-trimethoxybenzyl)-4-[N-benzyloxycarbonyl-L-prolyl]piperazine, its optical isomers, isolated or in the form of a mixture, and its addition salts with a pharmaceutically acceptable acid.

11. Process for the preparation according to claim 1 of the compound that is 1-(2,3,4-trimethoxybenzyl)-4-[S-benzyl-L-cysteinyl]piperazine, its optical isomers, isolated or in the form of a mixture, and its addition salts with a pharmaceutically acceptable acid.

12. Process for the preparation according to claim 1 of the compound that is 1-(2,3,4-trimethoxybenzyl)-4-(L-arginyl)piperazine, its optical isomers, isolated or in the form of a mixture, and its addition salts with a pharmaceutically acceptable acid.

13. Process for the preparation according to claim 1 of the compound that is 1-(2,3,4-trimethoxybenzyl)-4-[$N_\alpha$,$N_\delta$,$N_\omega$-tribenzyloxycarbonyl-L-arginyl]piperazine, its optical isomers, isolated or in the form of a mixture, and its addition salts with a pharmaceutically acceptable acid.

14. Process for the preparation according to claim 1 of the compound that is 1-(2,3,4-trimethoxybenzyl)-4-[2-amino-4,4-dicarboxybutyryl]piperazine, its optical isomers, isolated or in the form of a mixture, and its addition salts with a pharmaceutically acceptable acid.

15. Process for the preparation of a pharmaceutical composition containing as active ingredient at least one compound prepared according to one of claims 1 to 14 in combination with one or more pharmaceutically acceptable excipients or carriers.

16. Process for the preparation according to claim 15 of a pharmaceutical composition for use in the treatment and prevention of cerebral ischaemia, cerebral vascular hypoxia, cerebral vascular anoxia and cerebral oedema, in the treatment of cerebral vascular accidents, cerebral trauma, encephalopathies, neurodegenerative diseases and disorders associated with ageing, and also in the treatment and prevention of ischaemia of the peripheral type, in cardiology: myocardial ischaemia and coronary ischaemia and their various clinical manifestations: angina pectoris, myocardial infarct, rhythm disorders, vascular spasm, cardiac insufficiency, and also in ophthalmology and in otorhinolaryngology in the case of vascular chorio-retinitis episodes, vertigo of vascular origin, Menière's vertigo or other vertigos of inner ear origin.

**Patentansprüche**

**Patentansprüche für folgende Vertragsstaaten : AT, BE, CH, LI, DE, DK, FR, GB, IT, LU, NL, PT, SE, IE**

1. Verbindungen der allgemeinen Formel (I):

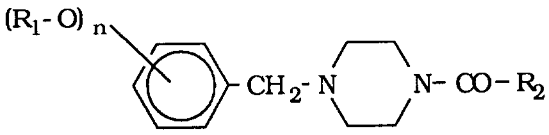

(I)

in der:

- n eine ganze Zahl mit den Werten 1, 2 oder 3,
- $R_1$ eine geradkettige oder verzweigte Alkylgruppe mit 1 bis 4 Kohlenstoffatomen,
- $R_2$: eine Gruppe

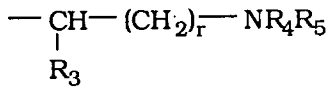

in der:

- r eine ganze Zahl mit den Werten 0, 1 oder 2,
- $R_3$:
  - a) ein Wasserstoffatom,
  - b) eine geradkettige oder verzweigte Alkylgruppe mit 1 bis 6 Kohlenstoffatomen, die gegebenenfalls substituiert ist durch
    * eine oder zwei Gruppen ausgewählt aus:
      - COOH, -CO-$R_6$, -CO-O-$R_6$, worin $R_6$ eine Gruppe ausgewählt aus gesättigten, ungesättigten oder polyungesättigten geradkettigen oder verzweigten Alkylgruppen mit 1 bis 12 Kohlenstoffatomen und -$(CH_2)_m$-Aryl, die gegebenenfalls substituiert ist und worin m eine ganze Zahl mit einem Wert von 0, 1, 2 oder 3 darstellt, bedeutet,
    * oder durch eine Gruppe ausgewählt aus:
      i) -OH, -O-$R_6$, -O-CO-$R_6$ oder -O-CO-O-$R_6$, worin $R_6$ die oben angegebenen Bedeutungen besitzt,
      ii) -$NR_7R_8$, worin $R_7$ und $R_8$, die gleichartig oder verschieden sein können, unabhängig voneinander jeweils ein Wasserstoffatom, eine geradkettige oder verzweigte Alkylgruppe mit 1 bis 6 Kohlenstoffatomen, -CO-$R_6$ oder -CO-O-$R_6$, worin $R_6$ die oben angegebenen Bedeutungen besitzt, und -$(CH_2)_m$-Aryl, die gegebenenfalls substituiert ist und worin m eine ganze Zahl mit den Werten von 0, 1, 2 oder 3 darstellt, bedeuten,
      iii) -CO-$NR_9R_{10}$, worin $R_9$ und $R_{10}$, die gleichartig oder verschieden sein können, die gleiche Bedeutung besitzen wie $R_6$, worin $R_6$ die oben angegebenen Bedeutungen besitzt, oder auch ein Wasserstoffatom bedeuten oder $R_9$ und $R_{10}$ gemeinsam mit dem Stickstoffatom, welches sie trägt, ein cyclisches System mit 5 bis 7 Ringgliedern bilden, das gegebenenfalls ein zweites Heteroatom ausgewählt aus Sauerstoff. Stickstoff und Schwefel, aufweist,
      iv) -SH oder -SeH,
      v) eine Gruppe -S-$R_6$, -Se-$R_6$, -S-CO-O-$R_6$, -S-S-$R_6$ oder -Se-Se-$R_6$, worin $R_6$ die oben angegebenen Bedeutungen besitzt,
      vi) eine Guanidinogruppe, die nicht substituiert oder mit 1 bis 2 Gruppen ausgewählt aus Nitrogruppen und Gruppen der Formel -CO-O-$R_6$, worin $R_6$ die oben angegebenen Bedeutungen besitzt, substituiert ist,
      vii) eine gegebenenfalls substituierte Indol- oder Imidazolgruppe,
  - c) eine Gruppe-$(CH_2)_m$-Phenyl, die gegebenenfalls substituiert ist und in der m eine ganze Zahl mit den Werten 0, 1, 2 oder 3 darstellt, wobei es sich versteht. daß $R_3$ keine Isobutylgruppe darstellt,
- $R_4$ ein Wasserstoffatom, eine geradkettige oder verzweigte Alkylgruppe mit 1 bis 6 Kohlenstoffatomen, eine gegebenenfalls substituierte Gruppe -$(CH_2)_p$-Aryl, worin p eine ganze Zahl mit den Werten von 0, 1, 2 oder 3 darstellt, oder eine Amidinogruppe, die nicht substituiert oder durch eine Nitrogruppe oder eine Gruppe der Formel -CO-O-$R_6$, worin $R_6$ die oben angegebenen Bedeutungen besitzt, substituiert ist,
  oder $R_4$ zusammen mit $R_3$ und den Atomen, an die sie gebunden sind, ein mono- oder bicyclisches System bilden, ausgewählt aus: Pyrrolidin, Piperidin, Perhydroindol, Indolin, 2-Azabicyclo[2.2.2]octan und 2-Azabicyclo[3.3.0]octan, wobei diese cyclischen Systeme gegebenenfalls durch eine oder mehrere Gruppen ausgewählt aus:

Hydroxylgruppen, Oxogruppen, geradkettige oder verzweigte Alkylgruppen mit 1 bis 6 Kohlenstoffatomen, geradkettige oder verzweigte Alkoxygruppen mit 1 bis 6 Kohlenstoffatomen substituiert sind, mit der Maßgabe, daß $R_3$ und $R_4$ nicht gemeinsam mit den Atomen, an die sie gebunden sind, einen durch eine Oxogruppe in $\alpha$-Stellung zum Stickstoffatom substituierten Pyrrolidinrest bilden,

- $R_5$:
  - ein Wasserstoffatom,
  - eine Gruppe $-(CH_2)_q$-Aryl, die gegebenenfalls substituiert ist und worin q eine ganze Zahl mit Werten von 0, 1, 2 oder 3 darstellt,
  - eine geradkettige oder verzweigte, gesättigte, ungesättigte oder polyungesättigte Alkylkette mit 1 bis 20 Kohlenstoffatomen, die gegebenenfalls durch eines oder mehrere Sauerstoffatome, Schwefelatome oder Stickstoffatome unterbrochen und gegebenenfalls durch eine oder mehrere Hydroxylgruppen, Aminogruppen, geradkettige oder verzweigte Alkylaminogruppen mit 1 bis 6 Kohlenstoffatomen oder geradkettige oder verzweigte Alkoxygruppen mit 1 bis 6 Kohlenstoffatomen substituiert ist,
  - eine Gruppe $-CO-R_{11}$ oder $-CO-O-R_{11}$, worin $R_{11}$:
    . eine geradkettige oder verzweigte, gesättigte, ungesättigte oder polyungesättigte Alkylkette mit 1 bis 20 Kohlenstoffatomen, die gegebenenfalls durch eines oder mehrere Sauerstoffatome, Schwefelatome oder Stickstoffatome unterbrochen sein kann und gegebenenfalls durch eine oder mehrere Hydroxylgruppen, Aminogruppen, geradkettige oder verzweigte Alkylaminogruppen mit 1 bis 6 Kohlenstoffatomen, geradkettige oder verzweigte Alkoxygruppen mit 1 bis 6 Kohlenstoffatomen, Guanidinogruppen oder mit 1 bis 2 Gruppen ausgewählt aus Nitrogruppen und Gruppen der Formel $-CO-O-R_6$, worin $R_6$ die oben angegebenen Bedeutungen besitzt, substituierte Guanidinogruppen substituiert ist,
    . eine gegebenenfalls substituierte Gruppe $-(CH_2)_q$-Aryl, worin q eine ganze Zahl mit Werten von 0, 1, 2 oder 3 darstellt.

  bedeuten,

wobei es sich versteht, daß, wenn nichts anderes angegeben ist:
- der Begriff "Aryl" für Phenyl- und Naphthylgruppen steht,
- der Begriff "substituiert" im Hinblick auf die Ausdrücke "$-(CH_2)_m$-Phenyl", "$-(CH_2)_m$-Aryl", "$-(CH_2)_p$-Aryl", "$-(CH_2)_q$-Aryl", "Imidazol" und "Indol" bedeutet, daß diese Gruppen am Ring durch eine oder mehrere Gruppen ausgewählt aus: Hydroxylgruppen, Halogenatome, Nitrogruppen, Trifluormethylgruppen, geradkettige oder verzweigte Alkylgruppen mit 1 bis 6 Kohlenstoffatomen, geradkettige oder verzweigte Alkoxygruppen mit 1 bis 6 Kohlenstoffatomen, $-(CH_2)_t$-Phenyl, $-O-(CH_2)_t$-Phenyl und $O-CO-O-(CH_2)_t$-Phenyl, worin t eine ganze Zahl mit Werten von 0, 1, 2 oder 3 darstellt, substituiert sein können,
- deren Isomere, Diastereoisomere, Epimere in isolierter Form oder in Form einer Mischung sowie gegebenenfalls deren Additionssalze mit einer pharmazeutisch annehmbaren Säure oder Base.

**2.** Verbindungen nach Anspruch 1, worin n den Wert 3 besitzt und $R_1$ eine Methylgruppe darstellt, wobei die in dieser Weise definierten drei Methoxygruppen in den Stellungen 2, 3 und 4 am aromatischen Kern des Benzylpiperazins stehen, was den Verbindungen der allgemeinen Formel (Iα) entspricht:

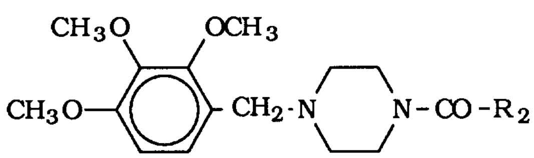

(I α)

in der $R_2$ die in Anspruch 1 angegebenen Bedeutungen besitzt;
- deren Isomere, Diastereoisomere und Epimere in isolierter Form oder in Form einer Mischung,
- und deren Additionssalze mit einer pharmazeutisch annehmbaren Säure oder Base.

**3.** Verbindungen nach Anspruch 1, worin $R_2$ eine Gruppe

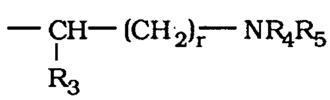

darstellt, wie sie in Anspruch 1 definiert worden ist, worin $R_3$ insbesondere eine geradkettige oder verzweigte Alkylgruppe mit 1 bis 6 Kohlenstoffatomen darstellt, die durch eine Gruppe -SH oder $-S-R_6$

substituiert ist, worin $R_6$ die in Anspruch 1 angegebenen Bedeutungen besitzt,
- deren Isomere, Diastereoisomere und Epimere in isolierter Form oder in Form einer Mischung
- und deren Additionssalze mit einer pharmazeutisch annehmbaren Säure oder Base.

**4.** Verbindungen nach Anspruch 1, worin
- n gleich 3 ist,
- $R_1$ eine Methylgruppe darstellt, wobei die in dieser Weise definierten drei Methoxygruppen in der 2-, 3- und 4-Stellung des aromatischen Kerns des Benzylpiperazins stehen,
- $R_2$ eine Gruppe der Formel

$$-\underset{\displaystyle R_3}{\underset{|}{CH}}-(CH_2)_r-NR_4R_5$$

darstellt, wie sie in Anspruch 1 definiert worden ist, worin $R_3$ eine geradkettige oder verzweigte Alkylgruppe mit 1 bis 6 Kohlenstoffatomen darstellt, die durch eine Gruppe -SH oder -S-$R_6$ substituiert ist, worin $R_6$ die in Anspruch 1 angegebenen Bedeutungen besitzt,
- deren Isomere, Diastereoisomere und Epimere in isolierter Form oder in Form einer Mischung
- und deren Additionssalze mit einer pharmazeutisch annehmbaren Säure oder Base.

**5.** Verbindungen nach Anspruch 1, worin $R_2$ eine Gruppe der Formel

$$-\underset{\displaystyle R_3}{\underset{|}{CH}}-(CH_2)_r-NR_4R_5$$

darstellt, wie sie in Anspruch 1 definiert worden ist, worin $R_3$ und $R_4$ gemeinsam mit den Atomen, an die sie gebunden sind, ein mono- oder bicyclisches System ausgewählt aus: Pyrrolidin, Piperidin, Perhydroindol, Indolin, 2-Azabicyclo[2.2.2]octan und 2-Azabicyclo[3.3.0]octan bilden, wobei diese cyclischen Systeme gegebenenfalls durch eine oder mehrere Gruppen ausgewählt aus:

Hydroxylgruppen, Oxogruppen, geradkettige oder verzweigte Alkylgruppen mit 1 bis 6 Kohlenstoffatomen und geradkettige oder verzweigte Alkoxygruppen mit 1 bis 6 Kohlenstoffatomen substituiert sind,

mit der Maßgabe, daß $R_3$ und $R_4$ nicht gemeinsam mit den Atomen, an die sie gebunden sind, einen in $\alpha$-Stellung zum Stickstoffatom durch eine Oxogruppe substituierten Pyrrolidinring bilden,
- deren Isomere, Diastereoisomere und Epimere in isolierter Form oder in Form einer Mischung
- und deren Additionssalze mit einer pharmazeutisch annehmbaren Säure oder Base.

**6.** Verbindungen nach Anspruch 1, worin $R_3$ eine geradkettige oder verzweigte Alkylgruppe mit 1 bis 6 Kohlenstoffatomen darstellt, die gegebenenfalls durch eine Guanidinogruppe oder eine mit 1 bis 2 Gruppen ausgewählt aus Nitrogruppen und Gruppen der Formel -CO-O-$R_6$, worin $R_6$ die in Anspruch 1 angegebenen Bedeutungen besitzt, substituierte Guanidinogruppe substituiert ist,
- deren Isomere, Diastereoisomere und Epimere in isolierter Form oder in Form einer Mischung
- und deren Additionssalze mit einer pharmazeutisch annehmbaren Säure oder Base.

**7.** Verbindung nach Anspruch 1, nämlich 1-(2,3,4-Trimethoxybenzyl)-4-[S-benzyl-N-benzyloxycarbonyl-L-cysteinyl]-piperazin, dessen optische Isomere in isolierter Form oder in Form einer Mischung und dessen Additionssalze mit einer pharmazeutisch annehmbaren Säure.

**8.** Verbindung nach Anspruch 1, nämlich 1-(2,3,4-Trimethoxybenzyl)-4-[N-benzyloxycarbonyl-L-alanyl]-piperazin, dessen optische Isomere in isolierter Form oder in Form einer Mischung und dessen Additionssalze mit einer pharmazeutisch annehmbaren Säure.

**9.** Verbindung nach Anspruch 1, nämlich 1-(2,3,4-Trimethoxybenzyl)-4-glycyl-piperazin und dessen Additionssalze mit einer pharmazeutisch annehmbaren Säure.

**10.** Verbindung nach Anspruch 1, nämlich 1-(2,3,4-Trimethoxybenzyl)-4-[N-benzyloxycarbonyl-L-propyl]-piperazin, dessen optische Isomere in isolierter Form oder in Form einer Mischung und dessen

Additionssalze mit einer pharmazeutisch annehmbaren Säure.

**11.** Verbindung nach Anspruch 1, nämlich 1-(2,3,4-Trimethoxybenzyl)-4-[S-benzyl-L-cysteinyl]-piperazin, dessen optische Isomere in isolierter Form oder in Form einer Mischung und dessen Additionssalze mit einer pharmazeutisch annehmbaren Säure.

**12.** Verbindung nach Anspruch 1, nämlich 1-(2,3,4-Trimethoxybenzyl)-4-(L-arginyl)-piperazin, dessen optische Isomere in isolierter Form oder in Form einer Mischung und dessen Additionssalze mit einer pharmazeutisch annehmbaren Säure.

**13.** Verbindung nach Anspruch 1, nämlich 1-(2,3,4-Trimethoxybenzyl)-4-[$N_\alpha$,$N_\delta$, $N_\omega$-tribenzyloxycarbonyl-L-arginyl]-piperazin, dessen optische Isomere in isolierter Form oder in Form einer Mischung und dessen Additionssalze mit einer pharmazeutisch annehmbaren Säure.

**14.** Verbindung nach Anspruch 1, nämlich 1-(2,3,4-Trimethoxybenzyl)-4-[2-amino-4,4-dicarboxybutyryl]-piperazin, dessen optische Isomere in isolierter Form oder in Form einer Mischung und dessen Additionssalze mit einer pharmazeutisch annehmbaren Säure.

**15.** Verfahren zur Herstellung der Verbindungen der Formel (I) nach Anspruch 1, **dadurch gekennzeichnet**, daß man eine Verbindung der Formel (II):

HOOC - $R'_2$ (II)

in der $R'_2$ die gleichen Bedeutungen besitzt, wie sie für $R_2$ in Anspruch 1 angegeben sind, mit der Maßgabe, daß wenn $R'_2$ eine Gruppe der Formel

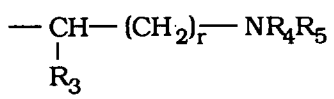

wie sie in Anspruch 1 definiert worden ist, darstellt und $R_4$ ein Wasserstoffatom bedeutet, $R_5$ eine Gruppe der Formel -CO-$R_{11}$ oder -CO-O-$R_{11}$, worin $R_{11}$ die in Anspruch 1 angegebenen Bedeutungen besitzt, darstellt,
unter Anwendung von Kupplungsmethoden, die über die Bildung eines aktivierten Esters (Hydroxysuccinimid, Hydroxybenzotriazol in Gegenwart von Dicyclohexylcarbodiimid) führen, mit einem (Alkoxybenzyl)-piperazin der Formel (III):

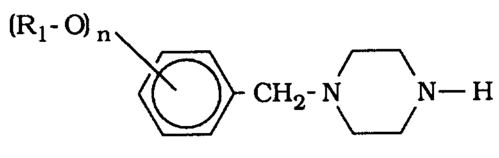

(III)

in der $R_1$ und n die bezüglich der Formel (I) angegebenen Bedeutungen besitzen, kondensiert zur Bildung einer Verbindung der Formel (I/a):

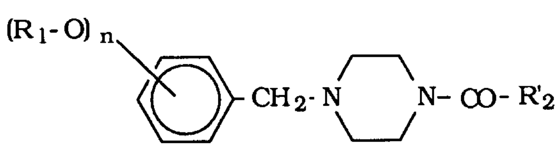

(I/a)

in der $R_1$, $R'_2$ und n die oben angegebenen Bedeutungen besitzen,
welche Verbindungen der Formel (I/a) dann, wenn $R_5$ eine Gruppe der Formel -CO-O-$R_{11}$ darstellt, gegebenenfalls Reaktionen zur Abspaltung von bei der Peptidsynthese verwendeten Schutzgruppen für die Aminogruppen unterworfen werden (Behandlung mit einer Säure oder katalytische Hydrierung in Abhängigkeit von der Art der Gruppe $R_{11}$) zur Bildung der Verbindungen der Formel (I) nach Anspruch

1, worin $R_5$ ein Wasserstoffatom darstellt, welche Verbindungen der Formel (I) nach Anspruch 1, worin $R_5$ ein Wasserstoffatom darstellt, anschließend gegebenenfalls einer Umsetzung mit einer Verbindung der Formel (IV):

HOOC - $R_{11}$ (IV)

worin $R_{11}$ die oben angegebenen Bedeutungen besitzt, unterworfen werden können zur Bildung einer Verbindung der Formel (I/b):

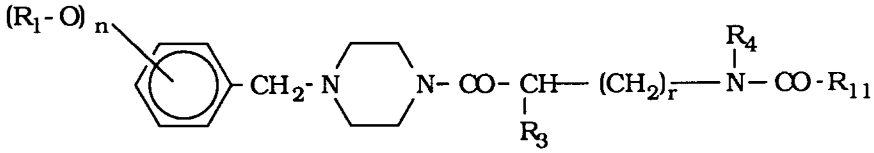

(I/b)

worin $R_1$, $R_3$, $R_4$, $R_{11}$ und n die oben angegebenen Bedeutungen besitzen,
einem Sonderfall der Verbindungen der Formel (I) nach Anspruch 1, worin $R_2$ eine Gruppe der Formel:

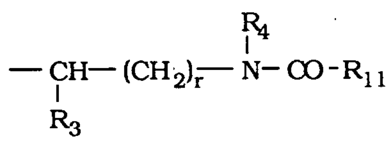

darstellt,
welche Verbindung der Formel (I) nach Anspruch 1 erforderlichenfalls gereinigt, gegebenenfalls in ihre verschiedenen optischen Isomeren aufgetrennt und gegebenenfalls entweder mit einer pharmazeutisch annehmbaren Säure oder, wenn dies möglich ist und wenn es erwünscht ist, mit einer pharmazeutisch annehmbaren Base in die Salze überführt werden können.

**16.** Pharmazeutische Zubereitung enthaltend als Wirkstoff mindestens eine Verbindung nach einem der Ansprüche 1 bis 14 in Kombination mit einem oder mehreren pharmazeutisch annehmbaren Trägermaterialien oder Bindemitteln.

**17.** Pharmazeutische Zubereitung nach Anspruch 16 zur Verwendung bei der Behandlung und der Vorbeugung der Zerebralischämie, der Zerebralgefäßhypoxie, der Zerebralgefäßanoxie und von Zerebralödemen, zur Behandlung von Zerebralgefäßvorfällen, Schädeltraumata, Enzephalopathien, neurodegenerativen Erkrankungen und Altersstörungen sowie zur Behandlung und der Vorbeugung der Ischämie peripheren Typs, in der Kardiologie: der Myokardischämie und der Koronarischämie und deren verschiedenen klinischen Ausprägungen: wie der Angina pectoris, des Myokardinfarkts, von Rhythmusstörungen. von Gefäßkrämpfen, von Herzinsuffizienzen, sowie in der Augenheilkunde und in der Hals-Nasen-Ohren-Heilkunde bei chorio-retinalen Gefäßvorfällen (Sigris-Hutchinson-Syndrom), von Schwindelzuständen mit Gefäßursprung, des Menière-Syndroms oder des Hörsturzes.

**Patentansprüche für folgende Vertragsstaaten : ES, GR**

**1.** Verfahren zur Herstellung von Verbindungen der allgemeinen Formel (I):

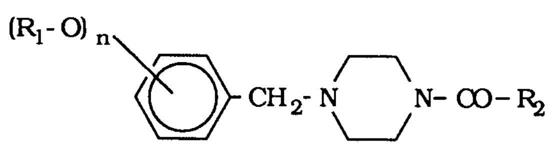

(I)

in der:
- n eine ganze Zahl mit den Werten 1, 2 oder 3,
- $R_1$ eine geradkettige oder verzweigte Alkylgruppe mit 1 bis 4 Kohlenstoffatomen,

- $R_2$: eine Gruppe

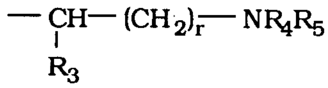

in der:
- r eine ganze Zahl mit den Werten 0, 1 oder 2,
- $R_3$:
  - a) ein Wasserstoffatom,
  - b) eine geradkettige oder verzweigte Alkylgruppe mit 1 bis 6 Kohlenstoffatomen, die gegebenenfalls substituiert ist durch
    * eine oder zwei Gruppen ausgewählt aus:
      - COOH, -CO-$R_6$, -CO-O-$R_6$, worin $R_6$ eine Gruppe ausgewählt aus gesättigten, ungesättigten oder polyungesättigten geradkettigen oder verzweigten Alkylgruppen mit 1 bis 12 Kohlenstoffatomen und -$(CH_2)_m$-Aryl, die gegebenenfalls substituiert ist und worin m eine ganze Zahl mit einem Wert von 0, 1, 2 oder 3 darstellt, bedeutet,
    * oder durch eine Gruppe ausgewählt aus:
      i) -OH, -O-$R_6$, -O-CO-$R_6$ oder -O-CO-O-$R_6$, worin $R_6$ die oben angegebenen Bedeutungen besitzt,
      ii) -$NR_7R_8$, worin $R_7$ und $R_8$, die gleichartig oder verschieden sein können, unabhängig voneinander jeweils ein Wasserstoffatom, eine geradkettige oder verzweigte Alkylgruppe mit 1 bis 6 Kohlenstoffatomen, -CO-$R_6$ oder -CO-O-$R_6$, worin $R_6$ die oben angegebenen Bedeutungen besitzt, und -$(CH_2)_m$-Aryl, die gegebenenfalls substituiert ist und worin meine ganze Zahl mit den Werten von 0, 1, 2 oder 3 darstellt, bedeuten,
      iii) -CO-$NR_9R_{10}$, worin $R_9$ und $R_{10}$, die gleichartig oder verschieden sein können, die gleiche Bedeutung besitzen wie $R_6$, worin $R_6$ die oben angegebenen Bedeutungen besitzt, oder auch ein Wasserstoffatom bedeuten oder $R_9$ und $R_{10}$ gemeinsam mit dem Stickstoffatom, welches sie trägt, ein cyclisches System mit 5 bis 7 Ringgliedern bilden, das gegebenenfalls ein zweites Heteroatom ausgewählt aus Sauerstoff, Stickstoff und Schwefel, aufweist,
      iv) -SH oder -SeH,
      v) eine Gruppe -S-$R_6$, -Se-$R_6$, -S-CO-O-$R_6$, -S-S-$R_6$ oder -Se-Se-$R_6$, worin $R_6$ die oben angegebenen Bedeutungen besitzt,
      vi) eine Guanidinogruppe, die nicht substituiert oder mit 1 bis 2 Gruppen ausgewählt aus Nitrogruppen und Gruppen der Formel -CO-O-$R_6$, worin $R_6$ die oben angegebenen Bedeutungen besitzt, substituiert ist,
      vii) eine gegebenenfalls substituierte Indol- oder Imidazolgruppe,
  - c) eine Gruppe -$(CH_2)_m$-Phenyl, die gegebenenfalls substituiert ist und in der m eine ganze Zahl mit den Werten 0, 1, 2 oder 3 darstellt,

  wobei es sich versteht, daß $R_3$ keine Isobutylgruppe darstellt,
- $R_4$ ein Wasserstoffatom, eine geradkettige oder verzweigte Alkylgruppe mit 1 bis 6 Kohlenstoffatomen, eine gegebenenfalls substituierte Gruppe -$(CH_2)_p$-Aryl, worin p eine ganze Zahl mit den Werten von 0, 1, 2 oder 3 darstellt, oder eine Amidinogruppe, die nicht substituiert oder durch eine Nitrogruppe oder eine Gruppe der Formel -CO-O-$R_6$, worin $R_6$ die oben angegebenen Bedeutungen besitzt, substituiert ist,

  oder $R_4$ zusammen mit $R_3$ und den Atomen, an die sie gebunden sind, ein mono- oder bicyclisches System bilden, ausgewählt aus: Pyrrolidin, Piperidin, Perhydroindol, Indolin, 2-Azabicyclo[2.2.2]octan und 2-Azabicyclo[3.3.0]octan, wobei diese cyclischen Systeme gegebenenfalls durch eine oder mehrere Gruppen ausgewählt aus:

  Hydroxylgruppen, Oxogruppen, geradkettige oder verzweigte Alkylgruppen mit 1 bis 6 Kohlenstoffatomen, geradkettige oder verzweigte Alkoxygruppen mit 1 bis 6 Kohlenstoffatomen

substituiert sind, mit der Maßgabe, daß $R_3$ und $R_4$ nicht gemeinsam mit den Atomen, an die sie gebunden sind, einen durch eine Oxogruppe in $\alpha$-Stellung zum Stickstoffatom substituierten Pyrrolidinrest bilden,
- $R_5$:
  - ein Wasserstoffatom,

- eine Gruppe -$(CH_2)_q$-Aryl, die gegebenenfalls substituiert ist und worin q eine ganze Zahl mit Werten von 0, 1, 2 oder 3 darstellt,
- eine geradkettige oder verzweigte, gesättigte, ungesättigte oder polyungesättigte Alkylkette mit 1 bis 20 Kohlenstoffatomen, die gegebenenfalls durch eines oder mehrere Sauerstoffatome, Schwefelatome oder Stickstoffatome unterbrochen und gegebenenfalls durch eine oder mehrere Hydroxylgruppen, Aminogruppen, geradkettige oder verzweigte Alkylaminogruppen mit 1 bis 6 Kohlenstoffatomen oder geradkettige oder verzweigte Alkoxygruppen mit 1 bis 6 Kohlenstoffatomen substituiert ist,
- eine Gruppe -CO-$R_{11}$ oder -CO-O-$R_{11}$, worin $R_{11}$:
  . eine geradkettige oder verzweigte, gesättigte, ungesättigte oder polyungesättigte Alkylkette mit 1 bis 20 Kohlenstoffatomen, die gegebenenfalls durch eines oder mehrere Sauerstoffatome, Schwefelatome oder Stickstoffatome unterbrochen sein kann und gegebenenfalls durch eine oder mehrere Hydroxylgruppen, Aminogruppen, geradkettige oder verzweigte Alkylaminogruppen mit 1 bis 6 Kohlenstoffatomen, geradkettige oder verzweigte Alkoxygruppen mit 1 bis 6 Kohlenstoffatomen, Guanidinogruppen oder mit 1 bis 2 Gruppen ausgewählt aus Nitrogruppen und Gruppen der Formel -CO-O-$R_6$, worin $R_6$ die oben angegebenen Bedeutungen besitzt, substituierte Guanidinogruppen substituiert ist,
  . eine gegebenenfalls substituierte Gruppe -$(CH_2)_q$-Aryl, worin q eine ganze Zahl mit Werten von 0, 1, 2 oder 3 darstellt,

  bedeuten,

wobei es sich versteht, daß, wenn nichts anderes angegeben ist:
- der Begriff "Aryl" für Phenyl- und Naphthylgruppen steht,
- der Begriff "substituiert" im Hinblick auf die Ausdrücke "-$(CH_2)_m$-Phenyl", "-$(CH_2)_m$-Aryl", "-$(CH_2)_p$-Aryl", "-$(CH_2)_q$-Aryl", "Imidazol" und "Indol" bedeutet, daß diese Gruppen am Ring durch eine oder mehrere Gruppen ausgewählt aus: Hydroxylgruppen, Halogenatome, Nitrogruppen, Trifluormethylgruppen, geradkettige oder verzweigte Alkylgruppen mit 1 bis 6 Kohlenstoffatomen, geradkettige oder verzweigte Alkoxygruppen mit 1 bis 6 Kohlenstoffatomen, -$(CH_2)_t$-Phenyl, -O-$(CH_2)_t$-Phenyl und O-CO-O-$(CH_2)_t$-Phenyl, worin t eine ganze Zahl mit Werten von 0, 1, 2 oder 3 darstellt, substituiert sein können,
- und von deren Isomeren, Diastereoisomeren, Epimeren in isolierter Form oder in Form einer Mischung sowie gegebenenfalls von deren Additionssalzen mit einer pharmazeutisch annehmbaren Säure oder Base,

**dadurch gekennzeichnet**, daß man eine Verbindung der Formel (II):

HOOC - $R'_2$ (II)

in der $R'_2$ die gleichen Bedeutungen besitzt, wie sie oben für $R_2$ bezüglich der Formel (I) angegeben sind, mit der Maßgabe, daß wenn $R'_2$ eine Gruppe der Formel

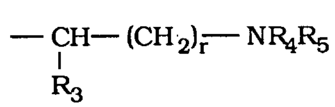

wie sie oben definiert worden ist, darstellt und $R_4$ ein Wasserstoffatom bedeutet, $R_5$ eine Gruppe der Formel -CO-$R_{11}$ oder -CO-O-$R_{11}$, worin $R_{11}$ die oben angegebenen Bedeutungen besitzt, darstellt, unter Anwendung von Kupplungsmethoden, die über die Bildung eines aktivierten Esters (Hydroxysuccinimid, Hydroxybenzotriazol in Gegenwart von Dicyclohexylcarbodiimid) führen, mit einem (Alkoxybenzyl)-piperazin der Formel (III):

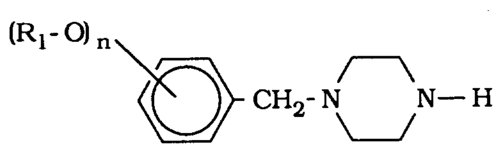 (III)

in der $R_1$ und n die bezüglich der Formel (I) angegebenen Bedeutungen besitzen, kondensiert zur Bildung einer Verbindung der Formel (I/a):

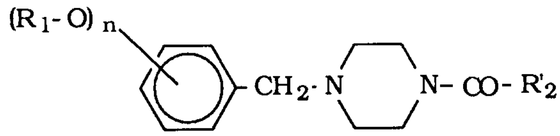

(I/a)

in der $R_1$, $R'_2$ und n die oben angegebenen Bedeutungen besitzen,
welche Verbindungen der Formel (I/a) dann, wenn $R_5$ eine Gruppe der Formel $-CO-O-R_{11}$ darstellt, gegebenenfalls Reaktionen zur Abspaltung von bei der Peptidsynthese verwendeten Schutzgruppen für die Aminogruppen unterworfen werden (Behandlung mit einer Säure oder katalytische Hydrierung in Abhängigkeit von der Art der Gruppe $R_{11}$) zur Bildung der Verbindungen der Formel (I), worin $R_5$ ein Wasserstoffatom darstellt, welche Verbindungen der Formel (I), worin $R_5$ ein Wasserstoffatom darstellt, anschließend gegebenenfalls einer Umsetzung mit einer Verbindung der Formel (IV):

$HOOC - R_{11}$ (IV)

worin $R_{11}$ die oben angegebenen Bedeutungen besitzt, unterworfen werden können zur Bildung einer Verbindung der Formel (I/b):

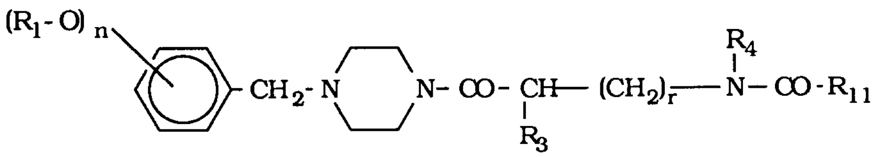

(I/b)

worin $R_1$, $R_3$, $R_4$, $R_{11}$ und n die oben angegebenen Bedeutungen besitzen,
einem Sonderfall der Verbindungen der Formel (I), worin $R_2$ eine Gruppe der Formel:

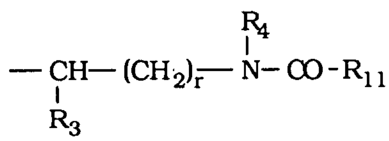

darstellt,
welche Verbindung der Formel (I) erforderlichenfalls gereinigt, gegebenenfalls in ihre verschiedenen optischen Isomeren aufgetrennt und gegebenenfalls entweder mit einer pharmazeutisch annehmbaren Säure oder, wenn dies möglich ist und wenn es erwünscht ist, mit einer pharmazeutisch annehmbaren Base in die Salze überführt werden können.

**2.** Verfahren nach Anspruch 1 zur Herstellung von Verbindungen, worin n den Wert 3 besitzt und $R_1$ eine Methylgruppe darstellt, wobei die in dieser Weise definierten drei Methoxygruppen in den Stellungen 2, 3 und 4 am aromatischen Kern des Benzylpiperazins stehen, was den Verbindungen der allgemeinen Formel (Iα) entspricht:

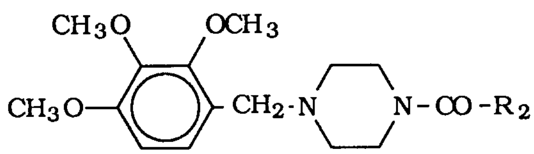

(I α)

in der $R_2$ die in Anspruch 1 angegebenen Bedeutungen besitzt;
- von deren Isomeren, Diastereoisomeren und Epimeren in isolierter Form oder in Form einer Mischung,
- und von deren Additionssalzen mit einer pharmazeutisch annehmbaren Säure oder Base.

**3.** Verfahren nach Anspruch 1 zur Herstellung von Verbindungen der Formel (I), worin $R_2$ eine Gruppe

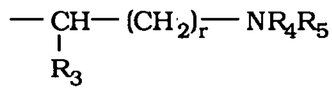

darstellt, wie sie in Anspruch 1 definiert worden ist, worin $R_3$ insbesondere eine geradkettige oder verzweigte Alkylgruppe mit 1 bis 6 Kohlenstoffatomen darstellt, die durch eine Gruppe -SH oder -S-$R_6$ substituiert ist, worin $R_6$ die in Anspruch 1 angegebenen Bedeutungen besitzt,

- von deren Isomeren, Diastereoisomeren und Epimeren in isolierter Form oder in Form einer Mischung
- und von deren Additionssalzen mit einer pharmazeutisch annehmbaren Säure oder Base.

**4.** Verfahren nach Anspruch 1 zur Herstellung von Verbindungen der Formel (I), worin

- n gleich 3 ist,
- $R_1$ eine Methylgruppe darstellt, wobei die in dieser Weise definierten drei Methoxygruppen in der 2-, 3- und 4-Stellung des aromatischen Kerns des Benzylpiperazins stehen,
- $R_2$ eine Gruppe der Formel

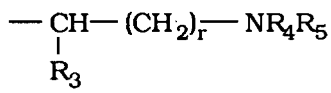

  darstellt, wie sie in Anspruch 1 definiert worden ist, worin $R_3$ eine geradkettige oder verzweigte Alkylgruppe mit 1 bis 6 Kohlenstoffatomen darstellt, die durch eine Gruppe -SH oder -S-$R_6$ substituiert ist, worin $R_6$ die in Anspruch 1 angegebenen Bedeutungen besitzt,
- von deren Isomeren, Diastereoisomeren und Epimeren in isolierter Form oder in Form einer Mischung
- und von deren Additionssalzen mit einer pharmazeutisch annehmbaren Säure oder Base.

**5.** Verfahren nach Anspruch 1 zur Herstellung von Verbindungen der Formel (I), worin $R_2$ eine Gruppe der Formel

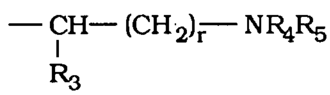

darstellt, wie sie in Anspruch 1 definiert worden ist, worin $R_3$ und $R_4$ gemeinsam mit den Atomen, an die sie gebunden sind, ein mono- oder bicyclisches System ausgewählt aus: Pyrrolidin, Piperidin, Perhydroindol, Indolin, 2-Azabicyclo[2.2.2]octan und 2-Azabicyclo[3.3.0]octan bilden, wobei diese cyclischen Systeme gegebenenfalls durch eine oder mehrere Gruppen ausgewählt aus:

Hydroxylgruppen, Oxogruppen, geradkettige oder verzweigte Alkylgruppen mit 1 bis 6 Kohlenstoffatomen und geradkettige oder verzweigte Alkoxygruppen mit 1 bis 6 Kohlenstoffatomen substituiert sind,

mit der Maßgabe, daß $R_3$ und $R_4$ nicht gemeinsam mit den Atomen, an die sie gebunden sind, einen in $\alpha$-Stellung zum Stickstoffatom durch eine Oxogruppe substituierten Pyrrolidinring bilden,

- von deren Isomeren, Diastereoisomeren und Epimeren in isolierter Form oder in Form einer Mischung
- und von deren Additionssalzen mit einer pharmazeutisch annehmbaren Säure oder Base.

**6.** Verfahren nach Anspruch 1 zur Herstellung von Verbindungen der Formel (I), worin $R_3$ eine geradkettige oder verzweigte Alkylgruppe mit 1 bis 6 Kohlenstoffatomen darstellt, die gegebenenfalls durch eine Guanidinogruppe oder eine mit 1 bis 2 Gruppen ausgewählt aus Nitrogruppen und Gruppen der Formel -CO-O-$R_6$, worin $R_6$ die in Anspruch 1 angegebenen Bedeutungen besitzt, substituierte Guanidinogruppe substituiert ist,

- von deren Isomeren, Diastereoisomeren und Epimeren in isolierter Form oder in Form einer Mischung
- und von deren Additionssalzen mit einer pharmazeutisch annehmbaren Säure oder Base.

**7.** Verfahren nach Anspruch 1 zur Herstellung der Verbindung, nämlich von 1-(2,3,4-Trimethoxybenzyl)-4-[S-benzyl-N-benzyloxycarbonyl-L-cysteinyl]-piperazin, von dessen optischen Isomeren in isolierter Form oder in Form einer Mischung und von dessen Additionssalzen mit einer pharmazeutisch annehmbaren Säure.

**8.** Verfahren nach Anspruch 1 zur Herstellung der Verbindung, nämlich von 1-(2,3,4-Trimethoxybenzyl)-4-[N-benzyloxycarbonyl-L-alanyl]-piperazin, von dessen optischen Isomeren in isolierter Form oder in Form einer Mischung und von dessen Additionssalzen mit einer pharmazeutisch annehmbaren Säure.

**9.** Verfahren nach Anspruch 1 zur Herstellung der Verbindung, nämlich von 1-(2,3,4-Trimethoxybenzyl)-4-glycyl-piperazin und von dessen Additionssalzen mit einer pharmazeutisch annehmbaren Säure.

**10.** Verfahren nach Anspruch 1 zur Herstellung der Verbindung, nämlich von 1-(2,3,4-Trimethoxybenzyl)-4-[N-benzyloxycarbonyl-L-propyl]-piperazin, von dessen optischen Isomeren in isolierter Form oder in Form einer Mischung und von dessen Additionssalzen mit einer pharmazeutisch annehmbaren Säure.

**11.** Verfahren nach Anspruch 1 zur Herstellung der Verbindung, nämlich von 1-(2,3,4-Trimethoxybenzyl)-4-[S-benzyl-L-cysteinyl]-piperazin, von dessen optischen Isomeren in isolierter Form oder in Form einer Mischung und von dessen Additionssalzen mit einer pharmazeutisch annehmbaren Säure.

**12.** Verfahren nach Anspruch 1 zur Herstellung der Verbindung, nämlich von 1-(2,3,4-Trimethoxybenzyl)-4-(L-arginyl)-piperazin, von dessen optischen Isomeren in isolierter Form oder in Form einer Mischung und von dessen Additionssalzen mit einer pharmazeutisch annehmbaren Säure.

**13.** Verfahren nach Anspruch 1 zur Herstellung der Verbindung, nämlich von 1-(2,3,4-Trimethoxybenzyl)-4-[$N_\alpha$, $N_\delta$, $N_\omega$-tribenzyloxycarbonyl-L-arginyl]-piperazin, von dessen optischen Isomeren in isolierter Form oder in Form einer Mischung und von dessen Additionssalzen mit einer pharmazeutisch annehmbaren Säure.

**14.** Verfahren nach Anspruch 1 zur Herstellung der Verbindung, nämlich von 1-(2,3,4-Trimethoxybenzyl)-4-[2-amino-4,4-dicarboxybutyryl]-piperazin, von dessen optischen Isomeren in isolierter Form oder in Form einer Mischung und von dessen Additionssalzen mit einer pharmazeutisch annehmbaren Säure.

**15.** Verfahren zur Herstellung einer pharmazeutischen Zubereitung enthaltend als Wirkstoff mindestens eine Verbindung hergestellt nach einem der Ansprüche 1 bis 14 in Kombination mit einem oder mehreren pharmazeutisch annehmbaren Trägermaterialien oder Bindemitteln.

**16.** Verfahren nach Anspruch 15 zur Herstellung einer pharmazeutischen Zubereitung zur Verwendung bei der Behandlung und der Vorbeugung der Zerebralischämie, der Zerebralgefäßhypoxie, der Zerebralgefäßanoxie und von Zerebralödemen, zur Behandlung von Zerebralgefäßvorfällen, Schädeltraumata, Enzephalopathien, neurodegenerativen Erkrankungen und Altersstörungen sowie zur Behandlung und der Vorbeugung der Ischämie peripheren Typs, in der Kardiologie: der Myokardischämie und der Koronarischämie und deren verschiedenen klinischen Ausprägungen: wie der Angina pectoris, des Myokardinfarkts, von Rhythmusstörungen, von Gefäßkrämpfen, von Herzinsuffizienzen, sowie in der Augenheilkunde und in der Hals-Nasen-Ohren-Heilkunde bei chorio-retinalen Gefäßvorfällen (Sigris-Hutchinson-Syndrom), von Schwindelzuständen mit Gefäßursprung, des Menière-Syndroms oder des Hörsturzes.